(19) Europäisches Patentamt
European Patent Office
Office européen des brevets

(11) Publication number: **0 251 417 B1**

(12) **EUROPEAN PATENT SPECIFICATION**

(45) Date of publication of patent specification: **14.04.93**

(51) Int. Cl.[5]: **C07D 211/58**, C07D 401/12, C07D 405/12, C07D 409/12, C07D 417/12, A61K 31/445

(21) Application number: **87201255.4**

(22) Date of filing: **01.07.87**

(54) **4-(aroylamino) piperidinebutanamide derivatives.**

(30) Priority: **03.07.86 US 882067**

(43) Date of publication of application:
**07.01.88 Bulletin 88/01**

(45) Publication of the grant of the patent:
**14.04.93 Bulletin 93/15**

(84) Designated Contracting States:
**AT BE CH DE ES FR GB GR IT LI LU NL SE**

(56) References cited:
**EP-A- 0 076 530**

**JOURNAL OF MEDICINAL CHEMISTRY, vol. 16, no. 7, July 1973, pages 782-786, Washington, D.C., US; R.A. STOKBROEKX et al.: "Synthetic antidiarrheal agents. 2,2-diphenyl-4-(4'-aryl-4'-hydroxypiperidino)-butyramides"**

(73) Proprietor: **JANSSEN PHARMACEUTICA N.V.**
**Turnhoutseweg 30**
**B-2340 Beerse(BE)**

(72) Inventor: **Van Daele, Georges Henri Paul**
**Kongostraat 156**
**B-2300 Turnhout(BE)**
Inventor: **Vlaeminck, Freddy François**
**Veldstraat 31**
**B-2418 Lille(BE)**
Inventor: **Sommen, François Maria**
**Langenberg 49**
**B-2323 Wortel(BE)**
Inventor: **De Cleyn, Michael Anna Jozef**
**Schuivenoord 30**
**B-2330 Merksplas(BE)**

EP 0 251 417 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is concerned with antidiarrheal agents, pharmaceutical compositions containing these agents and methods of treating warm-blooded animals suffering from diarrhea.

Diarrhea is one of the most common disorders. In many parts of the world, diarrhea produces more illness and kills more infants and children than all other diseases combined. Effective treatment of diarrhea may therefore save more lives and relieve more inconvenience than generally is recognized.

The present invention concerns the useful antidiarrheal properties of a number of 4-(aroylamino)-piperidinebutanamide derivatives and their use in the treatment of diarrhea.

Some of the 4-(aroylamino)piperidinebutanamide derivatives of the present invention are known from the Published European Patent Application No. 0,076,530 which corresponds to U.S. Application serial No. 362,814, while others are new.

In J. Med. Chem., 1973, Vol. 16, No. 7, pp. 782-786 there are described a number of 2,2-diphenyl-4-(4'-aryl-4'-hydroxypiperidino)butyramides as antidiarrheal agents.

In the U.S. Patent Nos. 3,647,805, 4,069,331 and 4,138,492 there are described a number of N-piperidinylbenzamides bearing a substituent in the 1-position of the piperidine ring as compounds being useful in the treatment of gastric ulcers, psychic disorders and migraine and as anti-emetics.

The present invention is concerned with the use for the manufacture of a medicament for the treatment of diarrhea, in particular in warm-blooded animals, of a compound having the formula:

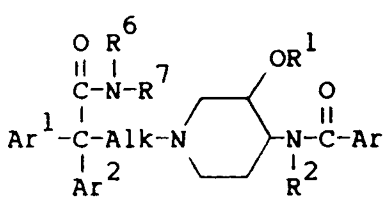

(I),

the N-oxide forms, the pharmaceutically acceptable acid-addition salts and possible stereoisomeric forms thereof, wherein

$R^1$ is hydrogen, $C_{1-6}$alkyl, aryl$C_{1-6}$alkyl, $C_{1-6}$alkylcarbonyl, amino$C_{1-6}$alkyl or mono- or di($C_{1-6}$alkyl)-amino$C_{1-6}$alkyl;

$R^2$ is hydrogen or $C_{1-6}$alkyl;

Ar is thienyl, halothienyl, furanyl, halofuranyl, pyridinyl, aminopyridinyl, thiazolyl, imidazolyl or a radical of formula

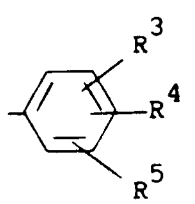

(a-1)

wherein $R^3$, $R^4$ and $R^5$ are each independently selected from the group consisting of hydrogen, $C_{1-6}$alkyl, $C_{1-6}$alkyloxy, halo, hydroxy, cyano, nitro, amino, mono- and di($C_{1-6}$alkyl)amino, aminocarbonyl, arylcarbonylamino, $C_{1-6}$alkylcarbonylamino, $C_{1-6}$alkylcarbonyl, $C_{1-6}$alkylcarbonyloxy, aminosulfonyl, $C_{1-6}$alkylsulfinyl,$C_{1-6}$lsulfonyl, $C_{1-6}$alkylthio, mercapto, $C_{3-6}$alkynyloxy, $C_{3-6}$alkenyloxy, aryl$C_{1-6}$alkyloxy, aryloxy and $C_{1-6}$alkyl substituted with up to 4 halo atoms;

Alk is $-CH_2-CH_2-$ or $-CH_2-CH(CH_3)-$;

$Ar^1$ and $Ar^2$ are, each independently, phenyl or halophenyl;

$R^6$ and $R^7$ are, each independently, hydrogen, $C_{1-6}$alkyl, phenylmethyl or 2-propenyl or $R^6$ and $R^7$ combined with the nitrogen atom bearing said $R^6$ and $R^7$ may form a pyrrolidinyl, piperidinyl, $C_{1-6}$alkylpiperidinyl, 4-morpholinyl or 2,6-di($C_{1-6}$alkyl)-4-morpholinyl radical;

wherein aryl is phenyl being optionally substituted with up to 3 substituents, i.e. 1, 2 or 3, each independently selected from the group consisting of halo, hydroxy, $C_{1-6}$alkyl, $C_{1-6}$alkyloxy, aminosulfonyl, $C_{1-6}$alkylcarbonyl, nitro, trifluoromethyl, amino, aminocarbonyl and phenylcarbonyl, said phenylcarbonyl being optionally substituted with up to 3 halo atoms; or thienyl being optionally substituted with halo or

$C_{1-6}$alkyl.

As used in the foregoing definitions the term halo is generic to fluoro, chloro, bromo and iodo; "$C_{1-6}$alkyl" is meant to include straight and branched saturated hydrocarbon radicals, having from 1 to 6 carbon atoms, such as, for example, methyl, ethyl, 1-methylethyl, 1,1-dimethylethyl, propyl, butyl, pentyl, hexyl and the like. "$C_{3-6}$alkenyl" is meant to include straight and branch chained hydrocarbon radicals containing one double bond and having from 3 to 6 carbon atoms such as, for example 3-propenyl, 2-butenyl and the like; "$C_{3-6}$alkynyl" is meant to include straight and branch chained hydrocarbon radicals having one triple bond and having from 3 to 6 carbon atoms such as, for example, propargyl, 2-butynyl, 3-butynyl, 2-pentynyl and the like. Preferably, in the said "$C_{3-6}$ alkenyl" or "$C_{3-6}$ alkynyl" radical being bound to a heteroatom, the carbon atom is saturated.

The said N-oxides of the compounds of formula (I) are meant to comprise those compounds of formula (I) wherein one or several nitrogen atoms are oxidated to the so called N-oxide form and in particular those N-oxides wherein the piperidine-nitrogen is N-oxidated.

The present invention is particularly concerned with the use for the manufacture of a medicament for the treatment of diarrhea, wherein, in the compound of formula (I), the substituents in the 3- and 4-position of the piperidine ring have the trans configuration.

A number of active ingredients of formula (I) are novel and have especially been developed for use in the manufacture of a medicament according to the present invention. These novel compounds constituting a further aspect of the present invention can be represented by the formula

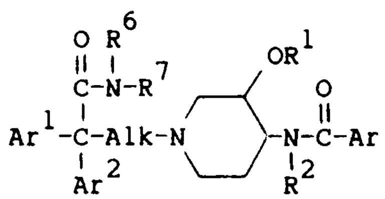

(I'),

the N-oxide forms, pharmaceutically acceptable acid-addition salts and stereochemically isomeric forms thereof, wherein $R^1$, $R^2$, $R^6$, $R^7$, Ar, $Ar^1$, $Ar^2$ and Alk have the previously described meaning provided that Ar is other than phenyl or 4-amino-5-chloro-2-methoxyphenyl when $R^6$ and $R^7$ are both methyl.

Preferred novel compounds are those compounds of formula (I') wherein Ar is thienyl, halothienyl, furanyl, halofuranyl, pyridinyl, aminopyridinyl, thiazolyl, imidazolyl or a radical of formula

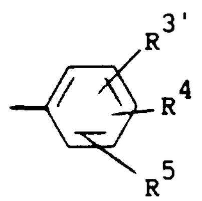

(a-2)

wherein $R^{3'}$ is $C_{3-6}$alkenyloxy, $C_{3-6}$alkynyloxy, aryl$C_{1-6}$alkyloxy, aryloxy or $C_{1-6}$alkyl substituted with up to 4 halo atoms, said $R^{3'}$ being substituted on either the ortho, para or meta position, and $R^4$ and $R^5$ have the previously described meanings.

More preferred novel compounds are those novel or preferred novel compounds wherein the substituents in the 3- and 4-position of the piperidine ring have the trans configuration.

Particularly preferred novel compounds are those more preferred novel compounds having one or more of the following particular substituents: Ar is thienyl, halothienyl, furanyl, halofuranyl, pyridinyl, aminopyridinyl, thiazolyl, imidazolyl or a radical of formula (a-2) wherein $R^{3'}$ is phenylmethoxy, phenoxy, propenyloxy or $C_{1-4}$alkyl substituted with up to 4 halo atoms and $R^4$ and $R^5$ are each independently hydrogen, $C_{1-4}$alkyl, $C_{1-4}$alkyloxy, halo, hydroxy, nitro, amino, $C_{1-4}$alkyl substituted with up to 4 halo atoms, phenylmethoxy, phenoxy or propenyloxy; $R^1$ is hydrogen or $C_{1-4}$alkyl; or $R^6$ and $R^7$ are, each independently hydrogen, $C_{1-4}$alkyl, phenylmethyl or 2-propenyl, or $R^6$ and $R^7$ combined with the nitrogen bearing said $R^6$ and $R^7$ may form a pyrrolidinyl, piperidinyl or 4-morpholinyl radical.

Especially preferred novel compounds are those particularly preferred novel compounds wherein Ar is a radical of formula (a-2) wherein $R^{3'}$ is trifluoromethyl substituted at the meta position and $R^4$ and $R^5$ are

each independently hydrogen, methyl, methoxy, halo, hydroxy, nitro, amino, trifluoromethyl, phenylmethoxy, phenoxy or propenyloxy.

The most preferred novel compounds within the invention are selected from the group consisting of trans-3-hydroxy-N,N,γ-trimethyl-α,α-diphenyl-4-[[3-(trifluoromethyl)benzoyl]amino]-1-piperidinebutanamide and the pharmaceutically acceptable acid-addition salts thereof.

In order to simplify the structural representations of the compounds of formula (I) and of certain precursors and intermediates thereof the

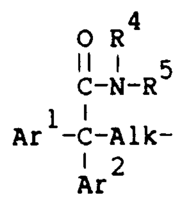

-radical will hereafter be represented by the symbol L.

The compounds of formula (I) can generally be prepared by the amidation reaction of an amine of formula

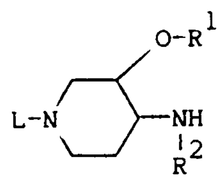 (II)

with a carboxylic acid of formula

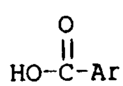 (III)

or a functional derivative thereof, such as, a halide, a symmetrical, a mixed or intramolecular anhydride, e.g. a cyclic anhydride of formula (III-a), or an activated ester, the latter also comprising internally activated esters such as, for example, the ester of formula (III-b).

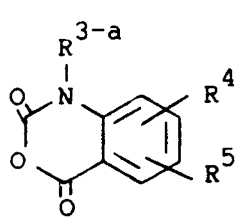 (III-a) 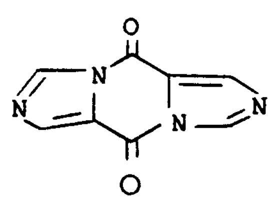 (III-b)

$R^{3-a}$ in formula (III-a) is hydrogen or mono$C_{1-6}$alkyl and $R^4$ and $R^5$ are as previously defined.

Said functional derivatives may be generated in situ, or, if desired be isolated and further purified before reacting them with the amine of formula (II). Functional derivatives may be prepared following art-known methods, for example, by reacting the carboxylic acid of formula (III) with thionyl chloride, phosphorous trichloride, polyphosphoric acid, phosphoryl chloride and the like, or by reacting the carboxylic acid of formula (III) with an acid halide e.g. acetyl chloride, ethyl carbonochloridate and the like.

Or, the compounds of formula (I) may be prepared by reacting (II) and (III), with a suitable reagent capable of forming amides, e.g., dicyclohexylcarbodiimide, 2-chloro-1-methylpyridinium iodide and the like. Said amidation reactions may conveniently be carried out by stirring a suitable reaction inert solvent such as, for

example, a halogenated hydrocarbon, e.g. dichloromethane, trichloromethane and the like, an aromatic hydrocarbon, e.g. methylbenzene and the like, an ether, e.g. 1,1'-oxybisethane, tetrahydrofuran and the like or a polar aprotic solvent, e.g. N,N-dimethylformamide, N,N-dimethylacetamide and the like. The addition of a suitable base such as, N,N-diethylethanamine may be appropriate. The water, the alcohol or the acid which is liberated during the course of the reaction is preferably removed from the reaction mixture following art-known procedures such as, for example, by azeotropical distillation, by complexation, by salt formation and the like methods.

The compounds of formula (I) wherein $R^1$ is hydrogen and wherein the substituents in the 3- and 4-positions of the piperidine ring have the trans configuration, said compounds being represented by the formula (I-a-1), can also be prepared by reacting a 7-oxa-3-azabicyclo[4.1.0]heptane of formula (IV) with an amide of formula (V). The compounds of formula (I-a-1) can further be O-alkylated or O-acylated following art-known procedures thus preparing the corresponding compounds of formula (I-a-2) wherein the substituents in the 3- and 4-positions of the piperidine ring have the trans configuration and wherein $R^1$ is other than hydrogen, said $R^1$ being represented by $R^{1-a}$.

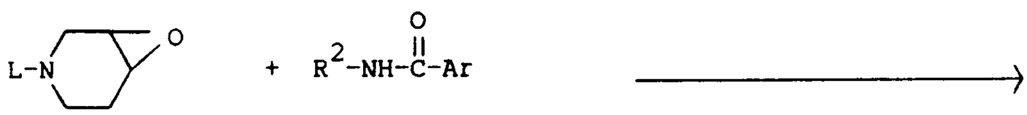

(IV) (V)

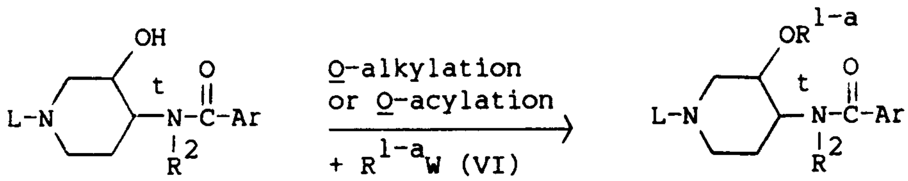


(I-a-1) (I-a-2)

In (I-a-1) and (I-a-2) the symbol "t" indicates that the substituents are in trans configuration.
In (VI) W represents an appropriate reactive leaving group such as, for example, halo, e.g. chloro, bromo or iodo, or a sulfonyloxy group, e.g. methylsulfonyloxy or (4-methylphenyl)sulfonyloxy.

The reaction of (IV) with (V) may conveniently be conducted by stirring and, if desired, heating the reactants in a suitable reaction-inert solvent, such as, for example, an alcohol, e.g., methanol, ethanol and the like.

The O-alkylation or O-acylation reactions are conveniently conducted in an inert organic solvent such as, for example, an aromatic hydrocarbon, e.g., benzene, methylbenzene, dimethylbenzene, and the like; a lower alkanol, e.g., methanol, ethanol, 1-butanol and the like; a ketone, e.g., 2-propane, 4-methyl-2-pentanone and the like; an ether, e.g., 1,4-dioxane, 1,1'-oxybisethane, tetrahydrofuran and the like; or a dipolar aprotic solvent e.g. N,N-dimethylformamide (DMF), N,N-dimethylacetamide (DMA), dimethyl sulfoxide (DMSO), 1-methyl-2-pyrrolidinone, and the like. An appropriate base such as, for example, an alkali metal carbonate, sodium hydride or an organic base such as, for example, N,N-diethylethanamine or N-(1-methylethyl)-2-propanamine may be utilized to pick up the acid which is liberated during the course of the reaction. In some instances the addition of a iodide salt, preferably an alkali metal iodide, is appropriate. Somewhat elevated temperatures may enhance the rate of the reaction.

The compounds of formula (I) wherein the substituents in the 3- and 4-positions of the piperidine ring have the cis configuration, said compounds being represented by the formula (I-b), can also be prepared by the reductive N-alkylation of a piperidine of formula (VII) with an amide of formula (V).

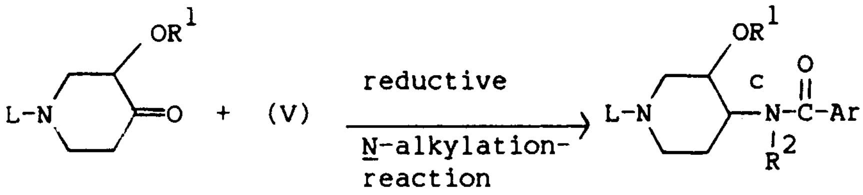


(VII) (I-b)

In (I-b) the symbol "c" indicates that the substituents are in cis configuration. Said reductive N-alkylation reaction may conveniently be carried out by catalytically hydrogenating a stirred and heated mixture of the reactants in a suitable reaction-inert organic solvent according to art-known catalytic hydrogenating procedures. Suitable solvents are, for example, water; alkanols, e.g. methanol, ethanol, 2-propanol and the like; cyclic ethers, e.g. 1,4-dioxane and the like; halogenated hydrocarbons, e.g. trichloromethane and the like; a polar aprotic solven e.g. N,N-dimethylformamide, dimethyl sulfoxide and the like; or a mixture of 2 or more of such solvents. The term "art-known catalytic hydrogenating procedures" means that the reaction is carried out under hydrogen atmosphere and in the presence of an appropriate catalyst such as, for example, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g., thiophene and the like.

The compounds of formula (I), can also be prepared by N-alkylating a piperidine of formula (VIII) with an intermediate of formula (IX) or with an ammonium salt of formula (X).

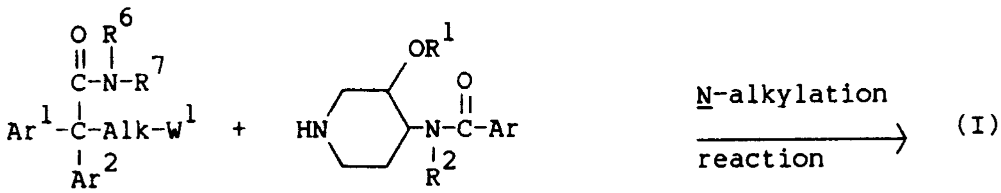


(IX) (VIII)

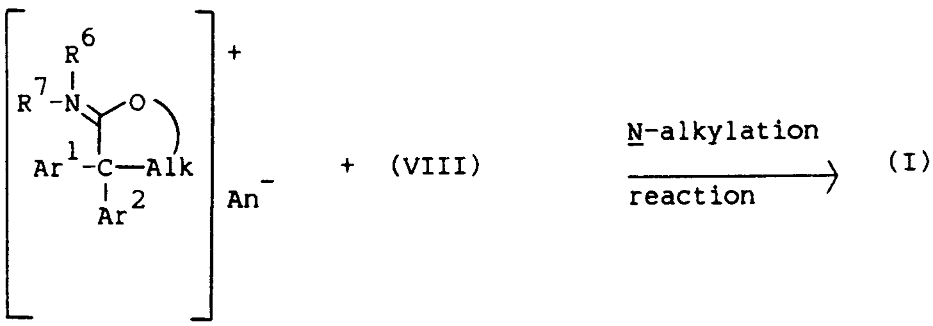


(X)

In (IX) $W^1$ represents an appropriate leaving group such as, for example, halo, e.g. chloro, bromo or iodo, or a sulfonyloxy group, e.g. methylsulfonyloxy or 4-methylsulfonyloxy. In (X) $An^-$ represents an appropriate anion such as, for example, a halide anion, e.g. chloride, bromide or iodide.

Said N-alkylation reactions are conveniently conducted in an inert organic solvent such as, for example, an aromatic hydrocarbon, e.g., benzene, methylbenzene, dimethylbenzene, and the like; an alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran and the like; a dipolar aprotic solvent e.g. N,N-dimethylformamide (DMF), N,N-dimethylacetamide (DMA), nitrobenzene, dimethyl sulfoxide (DMSO), 1-methyl-2-pyrrolidinone, and the like. The addition of an appropriate base such as, for example, an alkali metal carbonate or hydrogen carbonate or an organic base such as, for example, N,N-diethylethanamine or N-(1-methylethyl)-2-propanamine may be suited to pick up the acid which is liberated during the course of the reaction. In some instances the addition of a iodide salt, preferably an alkali metal iodide, is appropriate.

Somewhat elevated temperatures may enhance the rate of the reaction.

The compounds of formula (I) can alternatively be prepared by the reductive amination reaction of an appropriate ketone or aldehyde of formula L' = O (XI), said L' = O being a compound of formula L-H wherein two geminal hydrogen atoms are replaced by = O, with a piperidine of formula (VIII).

$$\underset{(XI)}{L'=O} + (VIII) \longrightarrow (I)$$

The compounds of formula (I) can also be converted into each other following art-known procedures of functional group transformation. Some examples thereof will be cited hereinafter.

The compounds of formula (I) having a nitro substituent can be converted into the corresponding amines by stirring and, if desired, heating the starting nitro-compounds in a hydrogen-containing medium in the presence of a suitable amount of an appropriate catalyst such as, for example, platinum-on-charcoal, palladium-on-charcoal, Raney-nickel and the like catalysts. Suitable solvents are, for example, methanol, ethanol and the like.

The hydrogen atom of the amino function of compounds of formula (I) may be replaced following art-known procedures such as, for example, N-alkylation, reductive N-alkylation, N-acylation and the like methods:

1) alkylcarbonyl, arylcarbonyl and the like groups may be introduced by reacting the starting amine with an appropriate carboxylic acid or a derivative thereof such as, for example, an acid halide, acid anhydride and the like.

2) alkyl groups may be introduced by reacting the starting amine with an alkanal or alkanone under a hydrogen atmosphere and in the presence of an appropriate catalyst such as, palladium-on-charcoal, platinum-on-charcoal and the like catalysts in suitable solvent such as, methanol, ethanol and the like. In order to prevent the undesired further hydrogenation of certain functional groups in the reactants and the reaction products it may be advantageous to add an appropriate catalyst-poison to the reaction mixture, e.g., thiophene and the like.

Compounds of formula (I) containing a hydroxy function may be converted into compounds of formula (I) containing a $C_{1-6}$alkylcarbonyloxy function by stirring the former with an appropriate acylating agent, e.g. an acid anhydride.

The compounds of formula (I) wherein Ar is phenyl substituent with phenylmethoxy may be converted into compounds of formula (I) wherein Ar is phenyl substituted with hydroxy following art-known catalytic hydrogenolysis procedures.

Halo atoms substituted on the benzamide moiety may be replaced by hydrogen following art-known hydrogenolysis procedures, i.e. by stirring and, if desired, heating the starting compounds in a suitable solvent under hydrogen atmosphere in the presence of an appropriate catalyst, e.g. palladium-on-charcoal and the like catalysts.

The compounds of formula (I) can be converted to the corresponding N-oxide forms following art-known procedures for converting a trivalent nitrogen to its N-oxide form. Said N-oxidation reaction may generally be carried out by reacting the starting material of formula (I) with an appropriate organic or inorganic peroxide. Appropriate inorganic peroxides comprise, for example, hydrogen peroxide, an alkali metal or earth alkaline metal peroxide, e.g. sodium peroxide, potassium peroxide, barium peroxide and the like; appropriate organic peroxides may comprise peroxy acids such as, for example, benzenecarboperoxoic acid or halo substituted benzenecarboperoxoic acid, e.g. 3-chlorobenzenecarboperoxoic acid and the like, peroxoalkanoic acids, e.g. peroxoacetic acid and the like, alkylhydroperoxides, e.g. t-butyl hydroperoxide and the like. If desired, said N-oxidation may be carried out in a suitable solvent such as for example, water, lower alkanols, e.g. methanol, ethanol, propanol, butanol and the like, hydrocarbons, e.g. benzene, methylbenzene, dimethylbenzene and the like, ketones, e.g. 2-propanone, 2-butanone and the like, halogenated hydrocarbons, e.g. dichloromethane, trichloromethane and the like, and mixtures of such solvents. In order to enhance the reaction rate, it may be appropriate to heat the reaction mixture.

In all of the foregoing and in the following preparations, the reaction products may be isolated from the reaction mixture and, if necessary, further purified according to methodologies generally known in the art.

The compounds of formula (I) having basic properties may be converted to their therapeutically active non-toxic acid addition salt forms by treatment with appropriate acids, such as, for example, inorganic acids,

such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

Conversely the salt form can be converted by treatment with alkali into the free base form.

Some of the intermediates and starting materials in the foregoing preparations are known compounds while other are novel. They may be prepared according to art-known methodologies of preparing said known or similarly known compounds. Some procedures for preparing such intermediates will be described hereinafter in more detail.

The intermediates of formula (II) can be derived from an appropriately substituted piperidine of formula (XII) by reacting the latter with a reagent of formula (IX) or (X), following the N-alkylation procedures described for the preparation of (I) starting from (X) and (VIII) and, subsequently, eliminating the protective group P in the thus obtained intermediate following art-known procedures, e.g., depending upon the case by hydrolysis in acidic or alkaline aqueous medium or by catalytic hydrogenation.

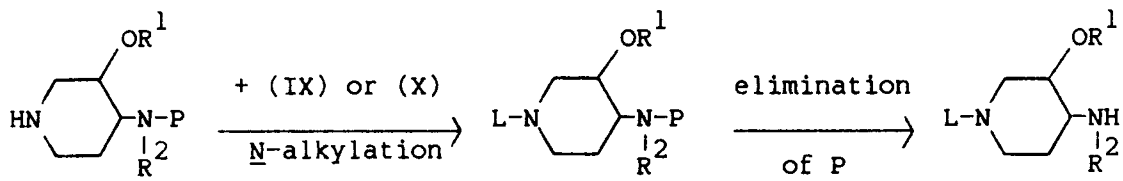


(XII) (XIII) (II)

The intermediates of formula (VIII) can be derived from an appropriately substituted piperidine formula of (XIV) by reacting the latter with a reagent of formula (III) or a functional derivative thereof, following the amidation procedures described for the preparation of (I) starting from (II) and (III), and subsequently eliminating the protective group P in the thus obtained intermediate following art-known procedures.

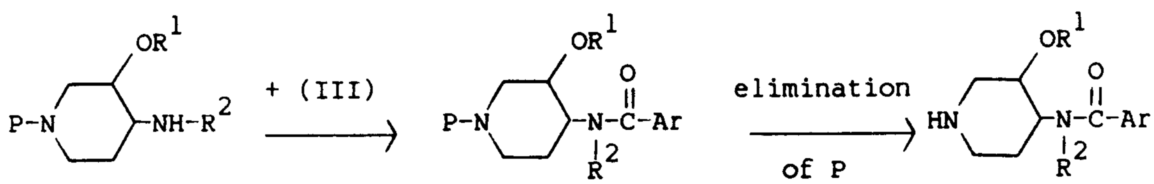


(XIV) (XV) (VIII)

In the foregoing and following reaction schemes P represents a suitable protective group which is readily removable by hydrogenation or hydrolysation, such as, phenylmethyl, $C_{1-4}$alkyloxycarbonyl and the like groups.

In general, the piperidines (XII), (XIV), (VII) and (IV) used as starting materials, can be prepared following procedures analogous to those described in the Published Euro. Pat. Appl. No. 0,076,530 which corresponds to U.S. Application Serial No. 362,814, and in Drug Development Research 8, 225-232 (1986).

The piperidines (XIV) wherein the substituents in the 3- and 4-position of the piperidine ring have the trans configuration, said piperidines being represented by formula (XIV-a), are preferably prepared from an appropriately substituted 7-oxa-3-azabicyclo[4.1.0]heptane, (XVI), by reacting the latter with an alkali metal azide (XVII) in a suitable reaction inert medium and by hydrogenating the thus obtained 4-azide-3-hydroxypiperidine, (XVIII), in the presence of a nobel catalyst, optionally after O-alkylating or O-acylating the hydroxy substituent with a reagent of formula (VI) following procedures described hereinabove for the preparation of (I-a-2) starting from (I-a-1) and (VI).

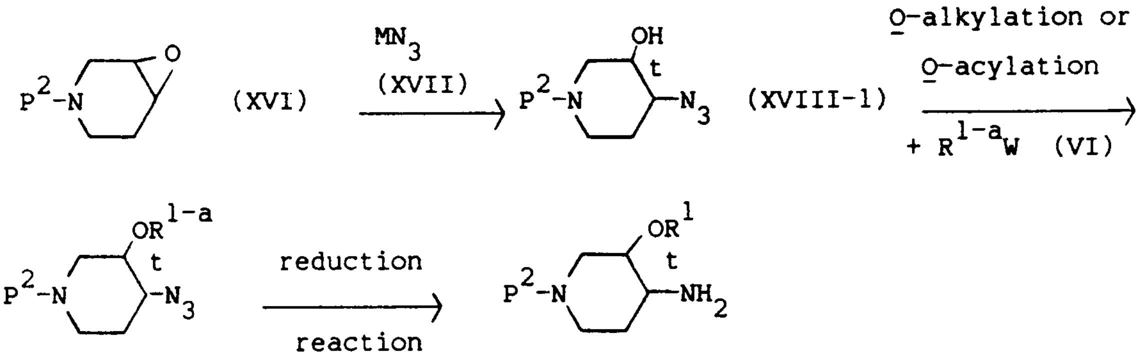


(XVIII-2) (XIV-a)

In formula (XVII) M is an appropriate alkali metal ion such as, for example, natrium, kalium and the like ions.

The compounds of formula (XIV), can easily be converted into the compounds of formula (XII) by introducing a protective group $P^1$ on the primary amine function, and selectively eliminating the protective group $P^2$ on the secondary amine function.

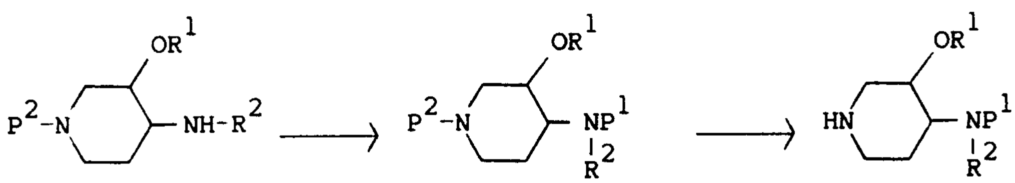

(XIV) (XVII) (XII)

The protective groups $P^1$ and $P^2$ should be selected so that $P^2$ can be eliminated without affecting $P^1$. Suitable protective groups are, for example, hydrogenolyzable groups as $P^1$ radicals, e.g. a phenylmethyl group and the like, and hydrolyzable groups as $P^2$ radicals, e.g., a $C_{1-6}$alkylcarbonyl group and the like.

The intermediates of formula (IX) and (X) and their preparations are described in U.S. Patent No. 3,714,159 and the the Journal of Medicinal Chemistry, 16, 782, (1973). All references mentioned hereinabove are incorporated herein by reference.

The intermediates of formula (VIII) wherein Ar is thienyl, halothienyl, furanyl, halofuranyl, pyridinyl, aminopyridinyl, thiazolyl, imidazolyl or a radical of formula

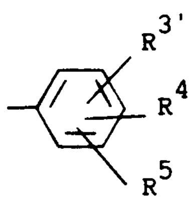
(a-2)

wherein $R^{3'}$is aryl$C_{1-6}$alkyloxy, aryloxy, $C_{3-6}$alkenyloxy, $C_{3-6}$alkynyloxy or $C_{1-6}$alkyl substituted with up to 4 halo atoms and $R^4$ and $R^5$ have the previously described meanings, said intermediates being represented by (VIII-a) are deemed to be novel intermediates, and as such they request an additional feature of the present invention. The compounds of formula (I) and some of the intermediates in this invention have one or more asymmetric carbon atom in their structure. Each of the chiral centers may be present in a R- or S-configuration, this R- and S-notation being in correspondence with the rules described in J. Org. Chem., 35, 2849-2867 (1970).

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, and enantiomers may be separated from each other by the selective crystallization of their diastereomeric salts

with optically active acids.

Pure stereochemically isomeric forms may also be derived from the corresponding pure stereochemically isomeric forms of the appropriate starting materials, provided that the reaction occurs stereospecifically.

It is evident that the cis and trans diastereomeric racemates may be further resolved into their optical isomers, cis(+), cis(-), trans(+) and trans(-) by the application of methodologies known to those skilled in the art.

In some compounds the stereochemical configuration is not experimentally determined. In those cases it is conventionally agreed to designate the stereochemically isomeric form which is first isolated as "A" or "X" and the second as "B" or "Y", without further reference to the actual stereochemical configuration.

Stereochemically isomeric forms of the compounds of formula (I) are naturally intended to be embraced within the scope of the invention.

The use of the compounds of formula (I), their N-oxide forms, pharmaceutically acceptable acid-addition salts and stereoisomeric forms thereof in the manufacture of a medicament according to the present invention is based on their useful antidiarrheal activity. This property is clearly evidence by the experimental data obtained in, for example, the "Ricinus Oil Test in Rats". The subject compounds are particularly attractive due to the strongly decreased and often absent undesired central effects. This can be demonstrated by the results of, for example, the "Tail Withdrawal Test in Rats". By virtue of their useful antidiarrheal activity, it is evident that the compounds of formula (I), their N-oxide forms, pharmaceutically acceptable acid addition salts and stereoisomeric forms can be used in the treatment of diarrhea. Due to the strongly decreased and often absent undesired central effects, they can particularly be useful in the treatment of diarrhea in subjects where medicines having undesired central effects can be harmful, for example, in the treatment of children and infants.

In view of their useful antidiarrheal properties, the subject compounds may be formulated into various pharmaceutical forms for administration purposes.

To prepare the pharmaceutical compositions of this invention, an effective amount of the particular compound, in base or acid-addition salt form as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deletorious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Those of skill in treating diarrhea could easily determine the effective amount from the test results presented hereinafter. In general it is contemplated that an effective amount would be from 0.001 mg/kg to 10 mg/kg body weight, more preferably from 0.005 mg/kg to 5 mg/kg body weight.

The following examples are intended to illustrate the scope of the present invention in all its aspects. Unless otherwise stated all parts therein are by weight.

## EXPERIMENTAL PART

### A. Preparation of Intermediates

#### Example 1

a) Gazeous carbonic dichloride was bubbled through a stirred solution of 16.1 parts of 2-amino-6-methoxybenzoic acid in 16.8 parts of concentrated hydrochloric acid and 140 parts of water for 2 hours. Gazeous nitrogen was bubbled through this mixture for 15 minutes. The product was filtered off, washed with water and dried, yielding 16.5 parts (88.9%) of 5-methoxy-2H-3,1-benzoxazine-2,4(1H)-dione (interm. 1).
b) To a stirred and cooled (<10 °C) solution of 9.65 parts of 5-methoxy-2H-3,1-benzoxazine-2,4(1H)-dione in 54 parts of N,N-dimethylformamide were added portionwise 2.64 parts of a sodium hydride dispersion 50%. After stirring for 1 hour in an ice bath, 7.81 parts of iodomethane were added dropwise at <15 °C. When the reaction mixture was solidified, 45 parts of N,N-dimethylformamide were added and a sodium hydride dispersion 50% was further added dropwise. Upon complete addition, stirring was continued overnight while the reaction mixture was allowed to reach room temperature. The reaction mixture was poured into ice water and 2,2'-oxybispropane was added. The precipitated product was filtered off, washed with water and dried, yielding 8.37 parts (80.7%) of 5-methoxy-1-methyl-2H-3,1-benzoxazine-2,4-(1H)-dione; mp. 215.8 °C (interm. 2)

#### Example 2

a) To a stirred emulsion of 53.8 parts of ethyl 7-oxa-3-azabicyclo[4,1,0]heptane-3-carboxylate, 17.6 parts of ethanol and 195 parts of water were added portionwise, during a 15 minutes-period, 28.6 parts of sodium azide while cooling in an ice bath. The mixture was warmed slowly to room temperature and stirring was continued overnight at room temperature. The aqueous phase was separated and extracted twice with dichloromethane. The combined organic phases were washed with a small amount of water, dried, filtered and evaporated, yielding 56.3 parts (82%) of a mixture of 80% of ethyl trans- 4-azido-3-hydroxy-1-piperidinecarboxylate (interm. 3) and 15% of ethyl trans-3-azido-4-hydroxy-1-piperidinecarboxylate.
b) To a stirred solution of 20.6 parts of 2-methyl-2-propanol, potassium salt in 54 parts of N,N-dimethylformamide was added dropwise a solution of 30.3 parts of a mixture of ethyl trans-4-azido-3-hydroxy-1-piperidinecarboxylate and ethyl trans-3-azido-4-hydroxy-1-piperidinecarboxylate in 45 parts of N,N,-dimethylformamide at a temperature below 20 °C (ice bath). Upon completion, stirring was continued for 1 hour at room temperature. 25.9 Parts of iodomethane were added dropwise at a temperature <10 °C. Upon completion, stirring was continued overnight at room temperature. The reaction mixture was poured into 400 parts of water. The product was extracted with trichloromethane. The extract was washed with a sodium chloride solution, dried, filtered and evaporated in vacuo. The residue was purified by column chromatography over silica gel using trichloromethane as eluent. The desired fractions were collected and the eluent was evaporated, yielding 15.6 parts (48.8%) of ethyl trans-4-azido-3-methoxy-1-piperidinecarboxylate as a residue (interm. 4).
c) A mixture of 15.6 parts of ethyl trans-4-azido-3-methoxy-1-piperidinecarboxylate and 200 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated in vacuo, yielding 13.8 parts (97.4%) of ethyl trans-4-amino-3-methoxy-1-piperidinecarboxylate as a residue (interm. 5).

#### Example 3

a-1) A mixture of 51.3 parts of ethyl 7-oxa-3-azabicyclo[4.1.0.]heptane-3-carboxylate, 36.4 parts of N-methylbenzenemethanamine and 480 parts of ethanol was stirred and refluxed for 42 hours. The reaction mixture was evaporated and the residue was taken up in a dilute hydrochloric acid solution. The aqueous phase was washed three times with 2,2'-oxybispropane and alkalized with a sodium hydroxide solution 50%. The product was extracted with dichloromethane. The extract was washed with water, dried, filtered

and evaporated. The residue was purified by column chromatography over silica gel using a mixture of trichloromethane and methanol (97:3 by volume) as eluent. The pure fractions were collected and the eluent was evaporated, yielding 45.9 parts (52.3%) of a mixture of ethyl trans-4-hydroxy-3-[methyl-(phenylmethyl)amino]-1-piperidinecar boxylate and ethyl trans-3-hydroxy-4-[methyl(phenylmethyl)amino]-1-piperdinecarboxylate (interm. 6) in the proportion of 62.3% and 32.9%. Intermediate 6 was also prepared according to the following procedure:

a-2) A mixture of 40 parts of ethyl trans-3-hydroxy-4-[(phenylmethyl)-amino]-1-piperidinecarboxylate, 15 parts of poly(oxymethylene), 2 parts of a solution of thiophene in methanol and 400 parts of methanol was hydrogenated at normal pressure and at room temperature with 4 parts of palladium-on-charcoal catalyst 10%. After the calculated amount of hydrogen was taken up, the catalyst was filtered off and the filtrate was evaporated. The residue was dissolved in trichloromethane. The organic layer was washed successively with a dilute ammonium hydroxide solution and water, dried, filtered and evaporated in vacuo. The residue was crystallized from 80 parts of acetonitrile. The product was filtered off and dried, yielding 32.7 parts (79.9%) of ethyl trans-3-hydroxy-4-[methyl(phenylmethyl)amino]-1-piperidinecarboxylate (interm. 6).

b) A mixture of 45.9 parts of ethyl trans-4-hydroxy-3-[methyl(phenylmethyl)amino]-1-piperidinecarboxylate and ethyl trans-3-hydroxy-4-[methyl(phenylmethyl)amino]-1-piperidinecarboxylate, 87.9 parts of potassium hydroxide and 576 parts of 2-propanol was stirred and refluxed for 4 hours. The reaction mixture was evaporated, water was added and evaporation was continued till all traces of 2-propanol were removed. The product was extracted three times with dichloromethane. The combined extracts were washed with a small amount of water, dried, filtered and evaporated. The residue was purified by column chromatography over silica gel using a mixture of trichloromethane and methanol, saturated with ammonia, (92:8 by volume) as eluent. The pure fractions were collected and the eluent was evaporated, yielding 32 parts of a mixture of trans-3-[methyl(phenylmethyl)amino]-4-piperidinol and trans-4-[methyl(phenylmethyl)amino]-3-piperidinol (interm. 7).

## Example 4

a) To a stirred solution of 10 parts of trans-3-methoxy-1-(phenylmethyl)-4-piperidinamine and 5.95 parts of N,N-diethylethanamine in 75 parts of trichloromethane was added dropwise a solution of 10.4 parts of 3-(trifluoromethyl)benzoyl chloride in 15 parts of N,N-diethylethanamine while cooling an ice bath. Upon completion, stirring was continued for 20 hours at room temperature. The mixture was washed twice with a sodium hydroxide solution 5% and once with water and then dried, filtered and evaporated in vacuo. After crystallization of the residue from 27 parts of methylbenzene, the product was filtered off, washed with 45 parts of methylbenzene and dried, yielding 14.4 parts (80.9%) of trans-N-[3-methoxy-1-(phenylmethyl)-4-piperidinyl]-3-(trifluoromethyl)benzamide; mp. 135.2 °C (interm. 8).

A mixture of 12 parts of trans-N-[3-methoxy-1-(phenylmethyl)-4-piperidinyl]-3-(trifluoromethyl)-benzamide and 200 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated in vacuo. The resiude was solidified on scratching in 2,2'-oxybispropane. The precipitated product was filtered off and dissolved in 135 parts of methylbenzene and 105 parts of 1,1'-oxybisethane. The supernatant liquid was decanted, washed twice with a dilute ammonium hydroxide solution and the product was extracted with methylbenzene. The aqueous phase was saturated with potassium carbonate and the product was extracted with methylbenzene. The combined organic layers were dried, filtered and evaporated in vacuo, yielding 6.43 parts (71%) of trans-N-(3-methoxy-4-piperidinyl)-3-(trifluoromethyl)benzamide;mp. 100.3 °C (interm. 9).

In a similar manner there were also prepared: cis-N-(3-methoxy-4-piperidinyl)-2-phenoxybenzamide; mp. 93.9 °C (interm 10);

cis-5-chloro-N-(3-methoxy-4-piperidinyl)-2-phenoxybenzamide ethanedioate (1:1); mp. 180.9 °C (interm. 11); and

trans-N-(3-hydroxy-4-piperidinyl)-3-(trilfuoromethyl)benzamide;mp. 164.8 °C (interm. 12).

## Example 5

a) To a stirred and cooled (5 °C) suspension of 81 parts of 4-chloro-2-methoxy-5-nitrobenzoic acid in 1350 parts of trichloromethane were added first 35.4 parts of N,N-diethylethanamine and then 38 parts of ethyl carbonochloridate at a temperature below 5 °C. The whole was stirred for 2 hours in an ice bath. A solution of 65 parts of ethyl cis-4-amino-3-methoxy-1-piperidinecarboxylate in 1125 parts of trichloromethane was added while the temperature was kept below 10° C. Stirring was continued first for 1 hour in an ice bath and then overnight at room temperature. The mixture was washed succesively once with water, twice with a sodium hydroxide solution 5% and three time with water, dried, filtered and evaporated. The residue was crystallized from acetonitrile. The product was filtered off and dried, yielding 100 parts (75%) of ethyl cis-4-[(4-chloro-2-methoxy-5-nitro-benzoyl)amino]-3-methoxy-1-piperidinecarboxylate; mp. 181.3° C (interm. 13).

b) A mixture of 90.5 parts of ethyl cis-4-[(4-chloro-2-methoxy-5-nitrobenzoyl)amino]-3-methoxy-1-piperidinecarboxylate, 3 parts of a solution of thiophene in methanol 4% and 400 parts of methanol was hydrogenated at normal pressure and at 50° C with 5 parts of platinum-on-charcoal catalyst 5%. After the calculated amount of hydrogen was taken up, the catalyst was filtered off and the filtrate was evaporated. The residue was crystallized from 2-propanol. The product was filtered off and dried, yielding 80 parts (94%) of ethyl cis-4-[5-amino-4-chloro-2-methoxybenzoyl)amino]-3-methoxy-1-piperidinecarboxylate; mp. 142.5° C (interm. 14).

c) A mixture of 81 parts of ethyl cis-4-[(5-amino-4-chloro-2-methoxybenzoyl)amino]-3-methoxy-1-piperidinecarboxylate, 122 parts of potassium hydroxide and 800 parts of 2-propanol was stirred for 6 hours at reflux temperature. The whole was stirred overnight at room temperature. The reaction mixture was evaporated. The residue was taken up in water and heated for a while. The mixture was evaporated again. The residue was taken up in water and the aqueous pahse was extracted twice with dichloromethane. The combined organic layers were washed with water, dried, filtered and evaporated. The residue was crystallized from acetonitrile. The product was filtered off and dried, yielding 58 parts (85%) of cis-5-amino-4-chloro-2-methoxy-N-(3-methoxy-4-piperidinyl)benzamide; mp. 191.8° C (interm. 15).

In a similar manner there were also prepared: trans-4-amino-5-chloro-N-(3-hydroxy-4-piperidinyl)-2-methoxybenzamide; mp. 185.2° C (interm. 16); and
trans-4-amino-5-chloro-2-methoxy-N-(3-methoxy-4-piperidinyl)benzamide; mp. 136.3° C (interm. 17).

Example 6

a) A mixture of 17.6 parts of trans-4-[(phenylmethyl)amino]-3-piperidinol, 27 parts of sodium carbonate and 680 parts of 4-methyl-2-pentanone was stirred and refluxed for 45 minutes using a water separator. After cooling, 33.8 parts of N-(dihydro-5-methyl-3,3-diphenyl-2(3H)-furanylidene)-N-methylmethanaminium bromide were added and stirring was continued for 24 hours at reflux. The mixture was cooled and washed with water. The organic layer was dried, filtered and evaporated. The residue was dissolved in 1,1'-oxybisethane and acidified with 2-propanol, saturated with hydrogen chloride. The liquid was decanted and the half solid precipitated product was dissolved in water and treated with ammonium hydroxide. The product was extracted with trichloromethane. The extract was dried, filtered and evaporated. The residue was purified by column chromatography over silica gel using a mixture of trichloromethane and methanol, saturated with ammonia, (90:10 by volume) as eluent. The desired fractions were collected and the eluent was evaporated. The residue was solidified on scratching in petroleumether. The product was filtered off and crystallized twice from acetonitrile. The product was filtered off and dried, yielding 18.8 parts (45%) of trans-3-hydroxy-N,N,γ-trimethyl-α,α-diphenyl-4-[(phenylmethyl)amino]-1-piperidinebutanamide; mp. 134.5° C (interm. 18).

b) A mixture of 63 parts of trans-3-hydroxy-N,N,γ-trimethyl-α,α-diphenyl-4-[(phenylmethyl)amino]-1-piperidinebutanamide and 485 parts of 2-methoxyethanol was hydrogenated at normal pressure and at 50° C with 5 parts of palladium-on-charcoal catalyst 10%. After the calculated amount of hydrogen was taken up, the cataylst was filtered off and the filtrate was evaporated with methylbenzene, yielding 45 parts (87.5%) of trans-4-amino-3-hydroxy-N,N,γ-trimethyl-α,α-diphenyl-1-piperdinebutanamide (interm. 19).

In a similar manner there were also prepared:
trans-4-amino-3-hydroxy-N,N-dimethyl-α,α-diphenyl-1-piperidinebutanamide (interm. 20);
trans-3-hydroxy-N,N,γ-trimethyl-4-(methylamino)-α,α-diphenyl-1-piperidinebutanamide; mp. 93.7° C (interm 21);
trans-1-[4-(4-amino-3-hydroxy-1-piperidinyl)-1-oxo-2,2-diphenylpentyl]-pyrrolidone as a residue (interm. 22);
trans-4-[4-(4-amino-3-hydroxy-1-piperidinyl)-1-oxo-2,2-diphenylpentyl]morpholine as a residue (interm. 23); and
cis-4-amino-3-hydroxy-N,N,γ-trimethyl-α,α-diphenyl-1-piperidinebutanamide as a residue (interm 24).

Example 7

a) A mixture of 29 parts of ethyl trans-4-amino-3-hydroxy-1-piperidinecarboxylate, 23.2 parts of (-)-[S-(R*,R*)]-2,3-dihydroxybutanedioc acid and 200 parts of ethanol was heated and the product was allowed to crystallize. After four crystallizations from ±320 parts of ethanol, the product was filtered off and dried, yielding 12 parts (21.3%) of (-)-ethyl (3B,trans)-4-amino-3-hydroxy-1-piperidinecarboxylate [S-(R*,R*)]-(2,3-dihydroxybutanedioate(1:1).monohydrate; mp. 148.8 °C

$$[\alpha]^{25}_{365} = -40.03° \text{ (c= 1\% in water) (interm. 25).}$$

b) A mixture of 11 parts of (-)-ethyl (3B,trans)-4-amino-3-hydroxy-1-piperidinecarboxylate [S-(R*,R*)]-2,3-dihydroxybutanedioate (1:1), 4.2 parts of benzaldehyde, 2 parts of a solution of thiophene in methanol 4%, 7.4 parts of potassium acetate and 200 parts of methanol was hydrogenated at normal pressure and at room temperature with 3 parts of platinum-on-charcoal catalyst 5%. 3.6 Parts of potassium hydroxide were added and after the calculated amount of hydrogen was taken up, the catalyst was filtered off and the filtrate was evaporated. The residue was converted into the hydrochloride salt in 2-propanol. The salt was filtered off and dried, yielding 10 parts (97.7%) of (+)-ethyl (3B,-trans)-3-hydroxy-4-[(phenylmethyl)-amino]-1-piperidinecarboxylate monohydrochloride; mp. 159.8 °C;

$$[\alpha]^{25}_{365} = +78.46° \text{ (c= 1\% in}$$

ethanol) (interm. 26).

c) A mixture of 8.5 parts of (+)-ethyl (3B,trans)-3-hydroxy-4-[(phenylmethyl)amino]-1-piperidinecarboxylate, 16.8 parts of potassium hydroxide and 120 parts of 2-propanol was stirred for 8 hours at reflux temperature. After evaporation, water was added to the residue and the solvent was evaporated again. The residue was taken up in water and the product was extracted with dichloromethane. The extract was washed with water, dried, filtered and evaporated. The residue was converted into the hydrochloride salt in 2-propanol and 2,2'-oxybispropane. The salt was filtered off and dried, yielding 1.35 parts (16.1%) of (+)-(3B,trans)-4-[(phenylmethyl)amino]-3-piperidinol dihydrochloride; mp. 196.6 °C;

$$[\alpha]^{25}_{365} = +92.01° \text{ (c= 1\% in ethanol) (interm. 27).}$$

d) A mixture of 8.8 parts of (+)-(3B,trans)-4-[(phenylmethyl)amino]-3-piperidinol, 6.3 parts of sodium carbonate and 200 parts of 4-methyl-2-pentanone was stirred and refluxed for 30 minutes using a water separator. After cooling, 16.9 parts of N-(dihydro-5-methyl-3,3-diphenyl-2(3H)-furanylidene)-N-methylmethanaminium bromide were added and stirring was continued for 17 hours at reflux. The mixture was filtered and the filtrate was evaporated. The residue was purified by column chromatography over silica gel using a mixture of trichloromethane and methanol, (95:5 by volume) as eluent. The pure fractions were collected and the eluent was evaporated. The residue was stirred in a mixture of acetonitrile and 2,2'-oxybispropane. The product was filtered off and dried, yielding 11.5 parts (55.0%) of (-)-(3B,trans)-3-hydroxy-N,N,γ-trimethyl-α,α-diphenyl-4-[(phenylmethyl)amino]-1-piperidinebutanamide (interm. 28).

e) A mixture of 9.5 parts of (-)-(3B,trans)-3-hydroxy-N,N,γ-trimethyl-α,α-diphenyl-4-[(phenylmethyl)amino-1-piperidinebutanamide and 200 parts of methanol was hydrogenated at normal pressure and at 50 °C with 2 parts of palladium-on-charcoal catalyst 10%. After the calculated amount of hydrogen was taken up, the catalyst was filtered of and the filtrate was evaporated, yielding 10 parts (100%) of (+)-(3B,trans)-4-amino-3-hydroxy-N,N,γ-trimethyl-α,α-diphenyl-1-piperidinebutanamide;

$$[\alpha]^{25}_{365} = +49.62° \text{ (c= 1\% in ethanol) (interm. 29).}$$

In a similar manner there was also prepared:
(-)-(3A,trans)-4-amino-3-hydroxy-N,N,γ-trimethyl-α,α-diphenyl-1-piperidinebutanamide,

$$[\alpha]^{25}_{589} = -13.68° \text{ (c= 1\% in ethanol) (interm. 30).}$$

Example 8

a) A mixture of 20 parts of (+)-(3B,trans)-4-[(phenylmethyl)amino]-3-piperidinol, 14.3 parts of sodium carbonate and 454 parts of 4-methyl-2-pentanone was stirred and refluxed for 30 minutes using a water separator. After cooling, 38.4 parts of N-(dihydro-5-methyl-3,3-diphenyl-2(3H)-furanylidene)-N-methylmethanaminium bromide were added and stirring was continued for 18 hours at reflux. The mixture was filtered and the filtrate was evaporated. The residue was purified by column chromatography over silica gel using a mixture of trichloromethane and methanol (95:5 by volume) as eluent. The desired fractions were collected and the eluent was evaporated. The residue was stirred in a mixture of acetonitrile and 2,2'-oxybispropane. The precipitated product was filtered off and crystallized twice from acetonitrile. The product was filtered off and dried, yielding 11.4 parts (23.9%) of (-)-[1(Y),3B, trans]-3-hydroxy-N,N,γ-trimethyl-α,α-diphenyl-4-[(phenylmethyl)amino]-1-piperidinebutanamide,

$$[\alpha]^{25}_{589} = +6.60° \text{ (c= 1\% in ethanol) (interm. 31).}$$

b) A mixture of 11 parts of (-)-[1(Y),3B,trans]-3-hydroxy-N,N,γ-trimethyl-α,α-diphenyl-4-[(phenylmethyl)-amino]-1-piperidinebutanamide and 120 parts of methanol was hydrogenated at normal pressure and at 50°C with 2 parts of palladium-on-charcoal catalyst 10%. After the calculated amount of hydrogen was taken up, the catalyst was filtered off and the filtrate was evaporated, yielding 9 parts (98.9%) of [1(Y),-3B,trans]-4-amino-3-hydroxy-N,N,γ-trimethyl-α,α-diphenyl-1-piperidinebutanamide as a residue (interm 32).

In a similar manner there was also prepared:
[1(Y),3A,trans]-4-amino-3-hydroxy-N,N,γ-trimethyl-α,α-diphenyl-1-piperidinebutanamide as a residue (interm. 33).

B. Preparation of Final Compounds

Example 9

To a stirred and cooled solution of 4 parts of trans-4-amino-3-hydroxy-N,N,γ-trimethyl-α,α-diphenyl-1-piperidinebutanamide in 120 parts of trichloromethane were added 1.26 parts of N,N-diethylethanamine. A solution of 2.3 parts of 3-(trifluoromethyl)benzoyl chloride in 75 parts of trichloromethane was added dropwise. Upon completion, the reaction mixture was stirred overnight at room temperature. A solution of sodium chloride in water was added. The separated organic layer was washed with water, dried, filtered and evaporated. The residue was taken up in 2,2'-oxybispropane. The precipitated product was filtered off and dried in vacuo at 60°C, yielding 4.9 parts (86.3%) of trans-3-hydroxy-N,N,γ-trimethyl-α,α-diphenyl-4-[[3-(trifluoromethyl)benzoyl]amino]-1-piperidinebutanamide; mp. 140.7°C (compound 1).

In a similar manner there were also prepared:

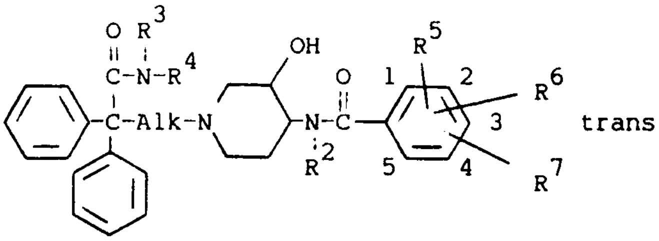

| No. | Alk | $R^3$ | $R^4$ | $R^2$ | $R^5$, $R^6$, $R^7$ | base/salt | mp.°C |
|---|---|---|---|---|---|---|---|
| 2 | $-CH_2-CH(CH_3)-$ | $CH_3$ | $CH_3$ | H | 2-Cl,3-Cl | base | 187.9 |
| 3 | $-CH_2-CH(CH_3)-$ | $CH_3$ | $CH_3$ | H | 2,3,4-$(OCH_3)_3$ | base | 197.2 |
| 4 | $-CH_2-CH(CH_3)-$ | $CH_3$ | $CH_3$ | H | 1-OH,2-Cl,3-Cl | base | 255.1 |
| 5 | $-CH_2-CH(CH_3)-$ | $CH_3$ | $CH_3$ | H | 1-Cl,5-Cl | base | 224.3 |
| 6 | $-CH_2-CH(CH_3)-$ | $CH_3$ | $CH_3$ | H | 1-OH,2-$NO_2$ | base | 247.4 |
| 7 | $-CH_2-CH(CH_3)-$ | $CH_3$ | $CH_3$ | H | 2-Cl,4-Cl | base | 203.0 |
| 8 | $-CH_2-CH(CH_3)-$ | $CH_3$ | $CH_3$ | H | 1-Cl,4-Cl | base | 225-230.7 |
| 9 | $-CH_2-CH_2-$ | $CH_3$ | $CH_3$ | H | 2-$CF_3$ | hemihydrate | 149.1 |
| 10 | $-CH_2-CH(CH_3)-$ | $CH_3$ | $CH_3$ | H | 2-Cl | base | 170.4 |
| 11 | $-CH_2-CH(CH_3)-$ | 1-pyrro-lidinyl | | H | 2-$CF_3$ | monohydrate | 129.8 |
| 12 | $-CH_2-CH(CH_3)-$ | $CH_3$ | $CH_3$ | $CH_3$ | 2-$CF_3$ | base | 138.7 |
| 13 | $-CH_2-CH(CH_3)-$ | 4-morpho-linyl | | H | 2-$CF_3$ | base | 117.3 |
| 14 | $-CH_2-CH(CH_3)-$ | $CH_3$ | $CH_3$ | H | 1-OH,3-Cl | base | 151.9 |
| 15 | $-CH_2-CH(CH_3)-$ | $CH_3$ | $CH_3$ | H | 1-OH,4-$NO_2$ | hemihydrate | 201.3 |
| 16 | $-CH_2-CH(CH_3)-$ | $CH_3$ | $CH_3$ | H | 1,3,5-$(CH_3)_3$ | base | 175.2 |
| 17 | $-CH_2-CH(CH_3)-$ | $CH_3$ | $CH_3$ | H | 1-$NO_2$,2-Cl | base | 212.0 |
| 18 | $-CH_2-CH(CH_3)-$ | $CH_3$ | $CH_3$ | H | 1-$NO_2$,3-Cl | base | 185.9 |
| 19 | $-CH_2-CH(CH_3)-$ | $CH_3$ | $CH_3$ | H | 1-$NO_2$,3-F | base | 194.5 |
| 20 | $-CH_2-CH(CH_3)-$ | $CH_3$ | $CH_3$ | H | 1-$NO_2$,2-$OCH_3$ | base | 214.0 |
| 21 | $-CH_2-CH(CH_3)-$ | $CH_3$ | $CH_3$ | H | 1-$NO_2$,4-$CH_3$ | base | 227.5 |
| 22 | $-CH_2-CH(CH_3)-$ | $CH_3$ | $CH_3$ | H | 3-CN | base | 148.7 |

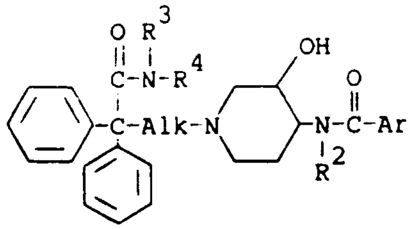

trans

| No. | Alk | $R^3$ | $R^4$ | $R^2$ | Ar | base/salt | mp.°C |
|---|---|---|---|---|---|---|---|
| 23 | $-CH_2-CH(CH_3)-$ | $CH_3$ | $CH_3$ | $CH_3$ | 3-pyridinyl | hemihydrate | 172.6 |
| | 2 3 | 3 | 3 | | 2 | | |
| 25 | $-CH_2-CH(CH_3)-$ | $CH_3$ | $CH_3$ | H | 5-Br-2-furanyl | base | 167.2 |
| 26 | $-CH_2-CH(CH_3)-$ | $CH_3$ | $CH_3$ | H | 2-thienyl | base | 181.3 |
| 27 | $-CH_2-CH(CH_3)-$ | $CH_3$ | $CH_3$ | H | 4-thiazolyl | base | 179.9 |

In a similar manner there was also prepared: cis-3-hydroxy-N,N,γ-trimethyl-α,α-diphenyl-4-[[3-(trifluoromethyl)-benzoyl]amino]-1-piperidinebutanamide ethanedioate(1:1); mp. 206. 3 ° C (compound 28).

Example 10

To a stirred and cooled (ice bath) solution of 4 parts of 2-(phenylmethoxy)benzoic acid in 90 parts of trichloromethane were added first 1.47 parts of N,N-diethylethanamine and then 1.6 parts of ethyl carbonochloridate at <5 ° C. After stirring for 1 hour in an ice bath, the thus obtained mixture was added dropwise to a cooled solution of 5.94 parts of trans-4-amino-3-hydroxy-N,N,γ-trimethyl-α,α-diphenyl-1-piperidinebutanamide in 90 parts of trichloromethane at a temperature below 5 ° C. Upon completion, stirring was continued overnight at room temperature. The organic layer was washed with water, a sodium carbonate solution in water and water, dried, filtered and evaporated. The residue was purified by column chromatography over silica gel using a mixture of trichloromethane and methanol (95:5 by volume) as eluent. The pure fractions were collected and the eluent was evaporated. The residue was solidified in 2,2'-oxybispropane. The product was filtered off and dried in vacuo at 60 ° C, yielding 3.7 parts (40.7%) of trans-3-hydroxy-N,N,γ-trimethyl-α,α-diphenyl-4-[[2-(phenylmethoxy)benzoyl]amino]-1-piperi dinebutanamide; mp. 149.0 ° C (compound 29).

In a similar manner there were also prepared:

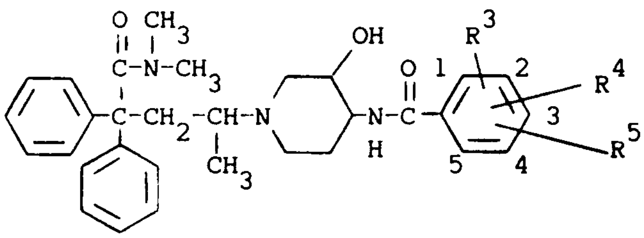

trans

| No. | $R^3$, $R^4$, $R^5$ | mp. °C |
|---|---|---|
| 30 | $1-OCH_3, 3-Cl$ | 210.3 |
| 31 | $1-OCH_3, 2-Cl, 4-Cl$ | 171.4 |
| 32 | $1-OCH_3, 4-SO_2-NH_2$ | 188.9 |
| 33 | $1-OCH_3, 3-NH-CH_3, 4-Cl$ | 191.8 |
| 34 | $2-CO-C_3H_7, 5-OCH_3$ | 123.2 |
| 35 | $1-OC_6H_5$ | 152.0 |
| 36 | $1-OCH_2-CH=CH_2, 3-Cl$ | 162.5 |
| 37 | $1-OCH_3, 3-SCH_3$ | 195.0 |
| 38 | $1-Br, 2-NO_2$ | 177.9 |
| 39 | $1-OCH_3, 3-NH-C(=O)-CH_3, 4-SCH_3$ | 178.8 |

In a similar manner there were also prepared: trans-4-[[4-(acetylamino)-2-(acetyloxy)benzoyl]amino-]-1-[4-(dimethylamino)-1-methyl-4-oxo-3,3-diphenylbutyl]-3-piperidinol acetate (ester); mp. 156.4° C (compound 40); and trans-3-hydroxy-N,N,γ-trimethyl-α,α-diphenyl-4-[(3-thienyl)carbonylamino]-1-piperidinebutanamide hemihydrate; mp. 194.4° C (compound 41).

Example 11

To a stirred solution of 3.95 parts of trans-4-amino-3-hydroxy-N,N,γ-trimethyl-α,α-diphenyl-1-piperidinebutanamide and 1.78 parts of 4-amino-5-cyano-2-hydroxybenzoic acid in 150 parts of trichloromethane were added 3.1 parts of N,N'-methanetetraylbis[cyclohexanamine] and stirring was continued over weekend at room temperature. The reaction mixture was acidified with an acetic acid solution in water. The separated organic layer was washed with water, dried, filtered and evaporated. The residue was taken up in acetonitrile and the precipitate was filtered off. The filtrate was purified by column chromatography over silica gel using a mixture of trichloromethane and methanol (90:10 by volume) as eluent. The pure fractions were collected and the eluent was evaporated. The residue was crystallized from acetonitrile. The product was filtered off and dried, yielding 0.55 parts (10%) of trans-4-[(4-amino-5-cyano-2-hydroxybenzoyl)amino]-3-hydroxy-N,N,γ-trimethyl-α,α-diphenyl-1-piperidinebutanamide monohydrate; mp. 211.4° C (compound 42).

Example 12

2.8 Parts of N,N-diethylethanamine were added to a solution of 1 part of 5H, 10H-diimidazo[1,5-a:1'5'd]-pyrazine-5,10-dione in 36 parts of N,N-dimethylformamide. The thus obtained suspension was added

dropwise to a stirred and heated (70 ° C) solution of 3.95 parts of trans-4-amino-3-hydroxy-N,N,γ-trimethyl-α,α-diphenyl-1-piperidinebutanamide in 18 parts of N,N-dimethylformamide. Upon complete addition, stirring was continued overnight at 70 ° C. After evaporation, the residue was purified by column chromatography over silica gel using a mixture of trichloromethane and methanol, saturated with ammonia. (90:10 by volume) as eluent. The pure fractions were collected and the eluent was evaporated. The residue was further purified by column chromatography (HPLC) over silica gel using a mixture of trichloromethane, methanol and methanol, saturated with ammonia, (90:9:1 by volume) as eluent. The pure fractions were collected and the eluent was evaporated. The residue was pulverised and evaporated again, yielding 1.13 parts (23%) of trans-3-hydroxy-4-[(1H-imidazol-5-yl)carbonylamino]-N,N,γ-trimethyl-α,α-diphenyl-1-piperidinebutanamide; mp. 155.0 ° C (compound 43).

Example 13

To a stirred and cooled (<5 ° C) solution of 3.95 parts of trans-4-amino-3-hydroxy-N,N,γ-trimethyl-α,α-diphenyl-1-piperidinebutanamide in 52 parts of trichloromethane was added dropwise a solution of 2.03 parts of 1-methyl-2H-3,1-benzoxazine-2,4(1H)dione in 48 parts of dichloromethane. Upon complete addition, the mixture was stirred for 32 hours at room temperature. The separated organic layer was washed with a sodium hydroxide solution 5% in water and water, dried, filtered and evaporated. The residue was purified by column chromatography over silica gel using a mixture of trichloromethane and methanol (96:4 by volume) as eluent. The pure fractions were collected and the eluent was evaporated. The residue was dissolved in trichloromethane. The organic layer was washed with a sodium hydroxide solution 5% in water and water, dried, filtered and evaporated. The residue was crystallized from acetonitrile. The product was filtered off and dried in vacuo at 60 ° C, yielding 0.5 parts (9.4%) of trans-3-hydroxy-N,N,γ-trimethyl-4-[[2-(methylamino)benzoyl]amino]-α,α-diphenyl-1-piperidinebutanamide; mp. 240.3 ° C (compound 44).

Example 14

To a stirred solution of 11.9 parts of trans-4-amino-3-hydroxy-N,N,γ-trimethyl-α,α-diphenyl-1-piperidinebutanamide in 180 parts of trichloromethane was added a solution of 5.8 parts of 5-methoxy-2H-3,1-benzoxazine-2,4(1H)-dione in 45 parts of N,N-dimethylformamide at 50 ° C. Stirring was continued for 2 hours at 50 ° C. After evaporation, the residue was suspended in water. The product was filtered off and purified by column chromatography over silica gel using a mixture of trichloromethane and methanol, saturated with ammonia, (95:5 by volume) as eluent. The first fraction was collected and the eluent was evaporated. The residue was suspended in 2,2'-oxybispropane. The product was filtered off and dried, yielding a first fraction of 7.60 parts of trans-4-[(2-amino-6-methoxybenzoyl)amino]-3-hydroxy-N,N,γ-trimethyl-α,α-diphenyl-1-piperidinebutanamide. The second fraction was collected and the eluent was evaporated. The residue was crystallized from acetonitrile. The product was filtered off and dried, yielding a second fraction of 1.23 parts of trans-4-[(2-amino-6-methoxybenzoyl)-amino]-3-hydroxy-N,N,γ-trimethyl-α,α-diphenyl-1-piperidinebutanamide. Total yield : 8.83 parts (54.1%) of trans-4-[(2-amino-6-methoxy-benzoyl)-amido]-3-hydroxy-N,N,γ-trimethyl-α,α-diphenyl-1-piperidine butanamide; mp. 175.1 ° C (compound 45).

In a similar manner there were also prepared:

trans-4-[(2-aminobenzoyl)amino]-3-hydroxy-N,N,γ-trimethyl-α,α-diphenyl-1-piperidinebutanamide monohydrate;mp. 164.5 ° C(compound 46);

trans-4-[(2-amino-5-chlorobenzoyl)amino]-3-hydroxy-N,N,γ-trimethyl-α,α-diphenyl-1-piperidinebutanamide; mp. 183.1 ° C (compound 47);

trans-4-[(2-amino-4-nitrobenzoyl)amino]-3-hydroxy-N,N,γ-trimethyl-α,α-diphenyl-1-piperidinebutanamide (compound 48); and

trans-3-hydroxy-4-[[2-methoxy-6-(methylamino)benzoyl]amino-N,N,γ-trimethyl-α,α-diphenyl-1-piperidinebutanamide; mp. 159.1 ° C (compound 49).

Example 15

A mixture of 4.5 parts of trans-4-amino-5-chloro-N-(3-hydroxy-4-piperidinyl)-2-methoxybenzamide, 4 parts of sodium carbonate, 0.1 parts of potassium iodide and 120 parts of 4-methyl-2-pentanone was stirred for 1 hour at reflux using a water separator. 5.95 Parts of N-(dihydro-5-methyl-3,3-diphenyl-2(3H)-furanylidene)-N-methylmethanaminium bromide were added and stirring was continued for 1 hour at reflux. The organic layer was washed successively with water, a sodium carbonate solution in water and water, dried, filtered and evaporated. The residue was purified by column chromatography over silica gel using a

mixture of trichloromethane and methanol, saturated with ammonia, (98:2 by volume) as eluent. The pure fractions were collected and the eluent was evaporated. The residue was dried in vacuo at 80 ° C, yielding 2.66 parts (30.6%) of trans-4-[(4-amino-5-chloro-2-methoxybenzoyl)amino]-3-hydroxy-N,N,γ-trimethyl-α,α-diphenyl-1-piperidinebutanamide; mp. 131.9 ° C (compound 50).

In a similar manner there was also prepared:

cis-4-[(5-amino-4-chloro-2-methoxybenzoyl)amino]-3-methoxy-N,N,γ-trimethyl-α,α-diphenyl-1-piperidinebutanamide; mp. 191.1 ° C (compound 51).

Example 16

A mixture of 3.0 parts of trans-N-(3-methoxy-4-piperidinyl)-3-(trifluoromethyl)benzamide,2.65 parts of sodium carbonate and 80 parts of 4-methyl-2-pentanone was stirred for 30 minutes at reflux temperature, using a water separator. After cooling, 3.96 parts of N-(dihydro-5-methyl-3,3-diphenyl-2(3H)-furanylidene)-N-methylmethanaminium bromide were added and stirring was continued for 5 hours at reflux. After cooling overnight at room temperature, the reaction mixture was washed twice with 50 parts of water, dried, filtered and evaporated in vacuo. The residue was purified by column chromatography over silica gel using a mixture of trichloromethane and methanol (95:5 by volume) as eluent. The pure fractions were collected and the eluent was evaporated. The residue was crystallized from 8 parts of acetonitrile. The product was filtered off and dried, yielding 1.3 parts (22.5%) of trans-3-methoxy-N,N,γ-trimethyl-α,α-diphenyl-4-[[3-(trifluoromethyl)benzoyl]amino]-1-piperidinebutanamide; mp. 197.8 ° C (compound 52).

In a similar manner there was also prepared:

trans-4-[(4-amino-5-chloro-2-methoxybenzoyl)amino]-3-methoxy-N,N,γ-trimethyl-α,α-diphenyl-1-piperidinebutanamide; mp. 184.8 ° C (compound 53).

Example 17

4.71 Parts of cis-4-amino-5-chloro-2-methoxy-N-(3-methoxy-4-piperidinyl)benzamide, 3.66 parts of sodium carbonate, 0.1 parts of potassium iodide and 120 parts of 4-methyl-2-pentanone was stirred and refluxed for 15 minutes using a water separator. 6 Parts of α-(2-bromopropyl)-N,N,-dimethyl-α-phenylbenzeneactamide were added and stirring was continued for 2.5 hours at reflux. Water was added. The organic layer was separated, washed with a sodium chloride solution, dried, filtered and evaporated. The residue was purified by column chromatography over silica gel using a mixture of trichloromethane and methanol, saturated with ammonia, (95:5 by volume) as eluent. The pure fractions were collected and the eluent was evaporated. The residue was suspended in 2,2'-oxybispropane. The product was filtered off and crystallized from acetonitrile, yielding 2.83 parts (31.8%) of cis-4-[(4-amino-5-chloro-2-methoxybenzoyl)amino]-3-methoxy-N,N,γ-trimethyl-α,α-diphenyl-1-piperidinebutanamide; mp. 198.3 ° C (compound 54).

Example 18

a) To a stirred and cooled solution of 3.1 parts of (+)-(3A,trans)-4-amino-3-hydroxy-N,N,γ-trimethyl-α,α-diphenyl-1-piperidinebutanamide in 14 parts of trichloromethane and 0.94 parts of N,N-diethylethanamine was added dropwise a solution of 1.8 parts of 3-(trifluoromethyl)benzoyl chloride in 75 parts of trichloromethane. Upon completion, stirring was continued overnight at room temperature. The reaction mixture was washed with a sodium carbonate solution in water and water. The separated organic layer was dried, filtered and evaporated. The residue was solidified in 2,2'-oxybispropane and crystallized from a mixture of acetonitrile and 2,2'-oxybispropane. The product was filtered off and dried, yielding 1.3 parts (29.3%) of (-)-(3A,trans)-3-hydroxy-N,N,γ-trimethyl-α,α-diphenyl-4-[[3-(trifluoromethyl)benzoyl]amino-1-piperidinebutanamide; mp. 197.7 ° C;

$$[\alpha]^{25}_{365} = -25.02° \text{ (c = 1\% in ethanol) (compound 55).}$$

b) 11.5 Parts of (-)-(3A,trans)-3-hydroxy-N,N,γ-trimethyl-α,α-diphenyl-4-[[3-trifluoromethyl)benzoyl]amino-1-piperidinebutanamide were crystallized three times from acetonitrile. The product was filtered off and dried, yielding 6.7 parts (59.0%) of (-)-[1(X),3A, trans]-3-hydroxy-N,N,γ-trimethyl-α,α-diphenyl-4-[[3-(trifluoromethyl)benzoyl]amino-1-piperidinebutanamide; mp. 215.1 ° C,

$$[\alpha]^{25}_{589} = -41.68° \text{ (c= 1\% in ethanol) (compound 56).}$$

In a similar manner there was also prepared:
(+)-(3B,trans)-3-hydroxy-N,N,γ-trimethyl-α,α-diphenyl-4-[[3-(trifluoromethyl)benzoyl]amino]-1-piperidinebutanamide; mp. 205.9 °C;

$$[\alpha]^{25}_{365} = +36.16° \text{ (c= 1\% in ethanol) (compound 57);}$$

and
(+)-[1(X),3B,trans]-3-hydroxy-N,N,γ-trimethyl-α,α-diphenyl-4-[[3-(trifluoromethyl)benzoyl]amino]-1-piperidinebutanamide; mp. 215.0 °C;

$$[\alpha]^{20}_{589} = +43.47° \text{ (c= 1\% in ethanol) (compound 58).}$$

Example 19

To a stirred and cooled (t<10 °C) solution of 9 parts of [1(Y),3B, trans]-4-amino-3-hydroxy-N,N,γ-trimethyl-α,α-diphenyl-1-piperidinebutanamide and 2.9 parts of N,N-diethylethanamine in 300 parts of trichloromethane was added dropwise a solution of 5.21 parts of 3-(trifluoromethyl)benzoyl chloride in 150 parts of trichloromethane. Upon completion, the reaction mixture was stirred for 3 hours at room temperature. The reaction mixture was washed with a sodium carbonate solution, dried, filtered and evaporated. The residue was purified by column chromatography over silica gel using a mixture of trichloromethane and methanol (95:5 by volume) as eluent. The pure fractions were collected and the eluent was evaporated. The residue was converted into the hyrochloride salt in 2-propanol. The salt was filtered off and dried for 48 hours in vacuo at 100 °C, yielding 4.56 parts (32.8%) of (-)-[1(Y),3B,trans]-3-hydroxy-N,N,γ-trimethyl-α,α-diphenyl-4-[[3-(trifluoromethyl)benzoyl]amino]-1-piperidinebutanamide monohydrochloride; mp. 209.6 °C;

$$[\alpha]^{20}_{589} = -37.62° \text{ (c= 1\% in ethanol) (compound 59).}$$

In a similar manner there was also prepared:
(+)-[1(Y),3A,trans]-3-hydroxy-N,N,γ-trimethyl-α,α-diphenyl-4-[[3-(trifluoromethyl)benzoyl]amino]-1-piperidinebutanamide; mp. 146.0 °C,

$$[\alpha]^{25}_{589} = +21.44° \text{ (c= 1\% in ethanol) (compound 60).}$$

Example 20

To a stirred solution of 2.72 parts of trans-4-[(2-amino-6-methoxy-benzoyl)amino]-3-hydroxy-N,N,γ-trimethyl-α,α-diphenyl-1-piperidinebutanamide in 20 parts of acetic acid were added 0.56 parts of acetic acid anhydride. After stirring overnight at room temperature, the reaction mixture was evaporated and the residue was purified by column chromatography over silica gel using a mixture of trichloromethane and methanol, saturated with ammonia, (95:5 by volume) as eluent. The pure fractions were collected and the eluent was evaporated. The residue was crystallized from 2,2'-oxybispropane. The product was filtered off and dried at 80 °C, yielding 2.45 parts (83.5%) of trans-4-[[2-(acetylamino)-6-methoxybenzoyl]amino]-3-hydroxy-N,N,γ-trimethyl-α,α-diphenyl-1-piperidinebutanamide; mp. 146.5 °C (compound 61).

In a similar manner there were also prepared:
cis-4-[[5-(acetylamino)-2-methoxybenzoyl]amino]-3-methoxy-N,N,γ-trimethyl-α,α-diphenyl-1-piperidinebutanamide; mp. 122.9 °C (compound 62);
trans-4-[[4-(acetylamino)-2-methoxybenzoyl]amino]-3-hydroxy-N,N,γ-trimethyl-α,α-diphenyl-1-piperidinebutanamide; mp. 193.8 °C (compound 63); and

trans-4-[[4-(acetylamino)-5-chloro-2-methoxybenzoyl]amino]-3-hydroxy-N,N,γ-trimethyl-α,α-diphenyl-1-piperidinebutanamide; mp. 147.2°C (compound 64).

Example 21

To a stirred solution of 6.52 parts of cis-4-[(5-amino-4-chloro-2-methoxybenzoyl)amino]-3-methoxy-N,N,γ-trimethyl-α,α-diphenyl-1-piperidinebutanamide in 195 parts of dichloromethane were added 2.6 parts of butanoyl chloride. After stirring for 15 minutes, 2.94 parts of N,N-diethylethanamine were added. The whole was stirred overnight at room temperature. The reaction mixture was washed successively with a sodium carbonate solution and water, dried, filtered and evaporated. The residue was purified by column chromatography over silica gel using a mixture of trichloromethane and methanol, saturated with ammonia, (95:5 by volume) as eluent. The pure fractions were collected and the eluent was evaporated. The residue was stirred in 2,2'-oxybispropane. The product was filtered off and dried, yielding 3.39 parts (46.5%) of cis-4-[[4-chloro-2-methoxy-5-[1-oxobutyl)amino]benzoyl]amino]-3-methoxy-N,N,γ-trimethyl-α,α-diphenyl-1-piperidinebutanamide; mp. 130.7°C (compound 65).

Example 22

A mixture of 2.3 parts of trans-4-[(4-amino-2-methoxybenzoyl)amino]-3-hydroxy-N,N,γ-trimethyl-α,α-diphenyl-1-piperidienbutanamide, 2 parts of poly(oxymethylene), 1 part of a solution of thiophene in methanol 4% and 120 parts of methanol was hydrogenated at normal pressure and at 50°C with 2 parts of palladium-on-charcoal catalyst 10%. After the calculated amount of hydrogen was taken up, the catalyst was filtered off and the filtrate was evaporated. The residue was crystallized from acetonitrile. The product was filtered off and dried in vacuo at 50°C, yielding 0.91 parts (39.7%) of trans-4-[[4-(dimethylamino)-2-methoxybenzoyl]amino]-3-hydroxy-N,N,γ-trimethyl-α,α-diphenyl-1-piperidinebutanamide; mp. 210.9°C (compound 66).

Example 23

A mixture of 4 parts of trans-4-[(4-fluoro-2-nitrobenzoyl)amino]-3-hydroxy-N,N,γ-trimethyl-α,α-diphenyl-1-piperidinebutanamide, 1 part of a solution of thiophene in methanol 4% and 200 parts of methanol was hydrogenated at normal pressure and at room temperature with 2 parts of platinum-on-charcoal catalyst 5%. After the calculated amount of hydrogen was taken up, the catalyst was filtered off and the filtrate was evaporated to dry. The residue was taken up in acetonitrile. The organic layer was evaporated again and the residue was crystallized from a mixture of acetonitrile and a few drops of water. The product was filtered off and dried, yielding 1.93 parts (51.8%) of trans-4-[(2-amino-4-fluorobenzoyl)amino]-3-hydroxy-N,N,γ-trimethyl-α,α-diphenyl-1-piperidinebutanamide monohydrate; mp. 127.0°C (compound 67).

In a similar manner there were also prepared:

trans-4-[(3-amino-2-hydroxybenzoyl)amino]-3-hydroxy-N,N,γ-trimethyl-α,α-diphenyl-1-piperidinebutanamide; mp. 157.2°C (compound 68);

trans-4-[(2-amino-3-chlorobenzoyl)amino]-3-hydroxy-N,N,γ-trimethyl-α,α-diphenyl-1-piperidinebutanamide; mp. 197.0°C (compound 69);

trans-4-[(2-amino-4-chlorobenzoyl)-amino]-3-hydroxy-N,N,γ-trimethyl-α,α-diphenyl-1-piperidinebutanamide monohydrate ; mp.130.9°C (compound 70);

trans-4-[(2-amino-5-methylbenzoyl)amino]-3-hydroxy-N,N,γ-trimethyl-α,α-diphenyl-1-piperidinebutanamide; mp. 216.8°C (compound 71);

trans-4-[(2,4-diaminobenzoyl)amino]-3-hydroxy-N,N-γ-trimethyl-α,α-diphenyl-1-piperidinebutanamide; mp. 136.1°C (compound 72); and

trans-4-[(2-amino-3-methoxybenzoyl)amino]-3-hydroxy-N,N,γ-trimethyl-α,α-diphenyl-1-piperidinebutanamide hemihydrate; mp. 169.5°C (compound 73).

Example 24

A mixture of 17.3 parts of cis-4-[(5-amino-4-chloro-2-methoxybenzoyl)amino]-3-methoxy-N,N,γ-trimethyl-α,α-diphenyl-1-piperidinebutanamide, 5 parts of calcium oxide and 250 parts of 2-methoxyethanol was hydrogenated at normal pressure and at 50°C with 2 parts of palladium-on-charcoal catalyst 10%. After the calculated amount of hydrogenation was taken up, the catalyst was filtered off and the filtrate was evaporated. The residue was purified by column chromatography over silica gel using a mixture of

trichloromethane and methanol, saturated with ammonia, (95:5 by volume) as eluent. The pure fractions were collected and the eluent was evaporated. The residue was crystallized from acetronitrile. The product was filtered off and dried, yielding 16.2 parts (100%) of cis-4-[(5-amino-2-methoxybenzoyl)amino]-3-methoxy-N,N,γ-trimethyl-α,α-diphenyl-1-piperidinebutanamide; mp. 189.0 °C (compound 74).

In a similar manner there were also prepared: trans-4-[(4-amino-2-methoxybenzoyl)amino]-3-hydroxy-N,N,γ-trimethyl-α,α-diphenyl-1-piperidinebutanamide; mp. 212.5 °C (compound 75); and cis-4-[(4-amino-2-methoxybenzoyl)amino]-3-hydroxy-N,N,γ-trimethyl-α,α-diphenyl-1-piperidinebutanamide; mp. 151.2 °C (compound 76).

Example 25

A mixture of 2.7 parts of trans-3-hydroxy-N,N,γ-trimethyl-α,α-diphenyl-4-[[2-(phenylmethoxy)benzoyl]-amino]-1-piperidinebutanamide and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was purified by column chromatography over silica gel using a mixture of trichloromethane and methanol (95:5 by volume) as eluent. The pure fractions were collected and the eluent was evaporated. The residue was taken up in methylbenzene and the solvent was evaporated again. The residue was suspended in a mixture of 2,2'-oxybispropane and a few drops of acetonitrile. The product was filtered off and dried in vacuo at 70 °C, yielding 1.3 parts (63.0%) of trans-3-hydroxy-4-[(2-hydroxybenzoyl)amino]-N,N,γ-trimethyl-α,α-diphenyl-1-piperidinebutanamide; mp. 154.3 °C (compound 77).

C. Pharmacological Examples

The useful pharmacological properties of the compounds of formula (I) and their pharmacological acceptable acid-addition salts can be demonstrated by the "Ricinus Oil Test" and by the "Tail Withdrawal Test".

Example 26

Ricinus Oil Test in Rats

Female Wistar rats were fasted overnight. Each animal was treated orally with a dose level of the compound to be tested. One hour thereafter, the animal received 1 ml of ricinus oil orally. Each animal was kept in an individual cage and 1 hour after the ricinus oil treatment, the presence or absence of diarrhea was noted. The $ED_{50}$ value was determined as that dose in mg/kg body weight, at which no diarrhea was present in 50% of the tested animals. Said $ED_{50}$-values for the compounds of the present invention can be found in the first column of Table 1.

Example 27

Tail Withdrawl Test

Male Wistar rats were fasted overnight. Each animal was treated orally with a dose level of the compound to be tested. The thus treated rats were put into individual restraining cages. At various time periods after administration, the lower 5 cm portion of the tail was immersed into a cup filled with water at a constant temperature of 55 °C. The typical tail withdrawal response was evaluated during a 10 seconds period after the immersion. $ED_{50}$ values in mg/kg body weight were determined as that dose of the test compound capable of suppressing in 50% of the tested animals the typical tail withdrawal response during a time period exceeding 10 seconds. Said $ED_{50}$ values obtained for the compounds of the present invention are gathered in Table 1 column two.

Table 1

| Comp. No. | Ricinus Oil Test $ED_{50}$ in mg/kg body weight | Tail Witdrawal Test $ED_{50}$ in mg/kg body weight |
|---|---|---|
| 50 | 2.5 | >40 |
| 53 | 2.5 | ~40 |
| 75 | 2.5 | >40 |
| 2 | 0.63 | >160 |
| 5 | 0.31 | ≧40 |
| 1 | 0.15 | >160 |
| 68 | 0.63 | >40 |
| 7 | 0.31 | >40 |
| 30 | 0.63 | ≧40 |
| 31 | 0.63 | ~40 |
| 47 | 0.08 | ~40 |
| 10 | 0.04 | >40 |
| 34 | <2.5 | >40 |
| 52 | 2.5 | 40 |
| 29 | ≦0.63 | ~40 |
| 77 | 0.16 | ≧40 |
| 64 | 2.5 | >40 |
| 38 | 2.5 | >40 |
| 17 | 0.63 | >40 |
| 69 | <0.63 | >40 |
| 19 | 1.25 | >40 |
| 67 | <0.04 | >40 |
| 70 | <0.63 | >40 |
| 21 | ≦0.63 | >40 |
| 57 | 0.31 | >160 |
| 55 | 1.0 | >160 |

D) Composition Examples

The following formulations exemplify typical pharmaceutical compositions in dosage unit form suitable for systemic administration to animal and human subjects in accordance with the instant invention. "Active ingredient" (A.I.) as used throughout these examples relates to a compound of formula (I) or a pharmaceutically acceptable acid addition salt thereof.

Example 28: ORAL DROPS

500 g of the A.I. was dissolved in 0.5 l of 2-hydroxypropanoic acid and 1.5 l of the polyethylene glycol at 60~80 ° C. After cooling to 30~40 ° C there were added 35 l of polyethylene glycol and the mixture was stirred well. Then there was added a solution of 1750 g of sodium saccharin in 2.5 l of purified water and while stirring there were added 2.5 l of coca flavor and polyethylene glycol q.s. to a volume of 50 l, providing an oral drop solution comprising 10 mg of the A.I. per ml. The resulting solution was filled into suitable containers.

Example 29 : ORAL SOLUTION

9 g of methyl 4-hydroxybenzoate and 1 g of propyl 4-hydroxy-benzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propane-triol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution comprising 20 mg of the active ingredient per teaspoonful (5 ml). The resulting solution was filled in suitable containers.

Example 30 : CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelating capsules, comprising each 20 mg of the active ingredient.

Example 31 : FILM-COATED TABLETS

Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90®) in about 200 ml of water. The wet powder mixture was sieved, dried and sieved again. Then there was added 100 g microcrystalline cellulose (Avicel®) and 15 g hydrogenated vegetable oil (Sterotex ®). The whole was mixed well and compressed into tablets, giving 10.000 tablets, each containing 10 mg of the active ingredient.

Coating

To a solution of 10 g methyl cellulose (Methocel 60 HG®) in 75 ml of denaturated ethanol there was added a solution of 5 g of ethyl cellulose (Ethocel 22 cps ®) in 150 ml of dichloromethane. Then there were added 75 ml of dichloromethane and 2.5 ml 1,2,3-propane-triol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated colour suspension (Opaspray K-1-2109®) and the whole was homogenated. The tablet cores were coated with the thus obtained mixture in a coating apparatus.

Example 32 : INJECTABLE SOLUTION

1.8 g methyl 4-hydroxybenzoate and 0.2 g propyl 4-hydroxybenzoate were dissolved in about 0.5 l of boiling water for injection. Aftercooling to about 50 ° C there were added while stirring 4 g lactic acid, 0.05 g propylene glycol and 4 g of the A.I.. The solution was cooled to room temperature and supplemented with water for injection q.s. ad l 1 volume, giving a solution of 4 mg A.I. per ml. The solution was sterilized by filtration (U.S.P. XVII p. 811) and filled in sterile containers.

Example 33 : SUPPOSITORIES

3 g A.I. was dissolved in a solution of 3 g 2,3-dihydroxybutanedioic acid in 25 ml polyethylene glycol 400. 12 g Surfactant (SPAN®) and triglycerides (Witepsol 555 ®) q.s. ad 300 g were molten together. The

latter mixture was mixed well with the former solution. The thus obtained mixture was poured into moulds at a temperature of 37~38 ° C to form 100 suppositories each containing 30 mg of the active ingredient.

## Claims

1. The use for the manufacture of a medicament for the treatment of diarrhea of a compound having the formula

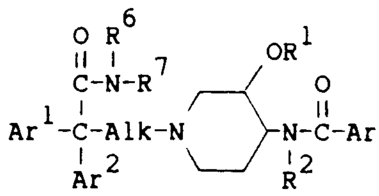

(I),

a N-oxide form, a pharmaceutically acceptable acid-addition salt or a possible stereoisomeric form thereof, wherein

$R^1$ is hydrogen, $C_{1-6}$alkyl, aryl$C_{1-6}$alkyl, $C_{1-6}$alkylcarbonyl, amino$C_{1-6}$alkyl or mono- or di-($C_{1-6}$alkyl)amino$C_{1-6}$alkyl;

$R^2$ is hydrogen or $C_{1-6}$alkyl;

Ar is thienyl, halothienyl, furanyl, halofuranyl, pyridinyl, aminopyridinyl, thiazolyl, imidazolyl or a radical of formula

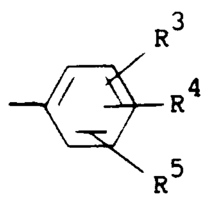

(a-1)

wherein $R^3$, $R^4$ and $R^5$ are each independently selected from the group consisting of hydrogen, $C_{1-6}$alkyl, $C_{1-6}$alkyloxy, halo, hydroxy, cyano, nitro, amino, mono- and di($C_{1-6}$alkyl)amino, aminocarbonyl, arylcarbonylamino, $C_{1-6}$alkylcarbonylamino, $C_{1-6}$alkylcarbonyl, $C_{1-6}$-alkylcarbonyloxy, aminosulfonyl, $C_{1-6}$alkylsulfinyl, $C_{1-6}$alkylsulfonyl, $C_{1-6}$alkylthio, mercapto, $C_{3-6}$alkynyloxy, $C_{3-6}$alkenyloxy, aryl$C_{1-6}$alkyloxy, aryloxy and $C_{1-6}$alkyl substituted with up to 4 halo atoms;

Alk is $-CH_2-CH_2-$ or $-CH_2-CH(CH_3)-$;

$Ar^1$ and $Ar^2$ are, each independently, phenyl or halophenyl;

$R^6$ and $R^7$ are, each independently, hydrogen, $C_{1-6}$alkyl, phenylmethyl or 2-propenyl or $R^6$ and $R^7$ combined with the nitrogen atom bearing said $R^6$ and $R^7$ may form a pyrrolidinyl, piperidinyl, $C_{1-6}$alkylpiperidinyl, 4-morpholinyl or 2,6-di($C_{1-6}$alkyl)-4-morpholinyl radical;

wherein aryl is phenyl being optionally substituted with up to 3 substituents each independently selected from halo, hydroxy, $C_{1-6}$alkyl, $C_{1-6}$alkyloxy, aminosulfonyl, $C_{1-6}$alkylcarbonyl, nitro, trifluoromethyl amino, aminocarbonyl and phenylcarbonyl said phenylcarbonyl being optionally substituted with up to 3 halo atoms; or thienyl being optionally substituted with halo or $C_{1-6}$alkyl.

2. The use according to claim 1 wherein the substituents in 3- and 4-position of the piperidine ring have the trans configuration.

**3.** A chemical compound having the formula

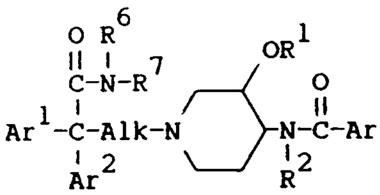

(I'),

a N-oxide form, a pharmaceutically acceptable acid-addition salt or a possible stereoisomeric form thereof, wherein

$R^1$, $R^2$, $R^6$, $R^7$, Ar, $Ar^1$, $Ar^2$ and Alk are as defined in claim 1, provided that Ar is other than phenyl or 4-amino-5-chloro-2-methoxyphenyl when $R^6$ and $R^7$ are both methyl.

**4.** A chemical compound according to claim 3 wherein Ar is thienyl, halothienyl, furanyl, halofuranyl, pyridinyl, aminopyridinyl, thiazolyl, imidazolyl or a radical of formula

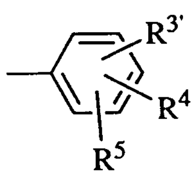

(a-2)

wherein $R^{3'}$ is aryl$C_{1-6}$alkyloxy, aryloxy, $C_{3-6}$alkenyloxy, $C_{3-6}$alkynyloxy or $C_{1-6}$alkyl substituted with up to 4 halo atoms.

**5.** A chemical compound according to any of claims 3 or 4 wherein the substituents in the 3- and 4-position of the piperidine ring have the trans configuration.

**6.** A chemical compound according to claim 5 wherein Ar is thienyl, halothienyl, furanyl, halofuranyl, pyridinyl, aminopyridinyl, thiazolyl, imidazolyl or a radical of formula (a-2) wherein $R^{3'}$ is phenylmethoxy, phenoxy, propenyloxy or $C_{1-4}$alkyl substituted with up to 4 halo atoms and $R^4$ and $R^5$ are each independently hydrogen. $C_{1-4}$alkyl, $C_{1-4}$alkyloxy, halo, hydroxy, nitro, amino, phenylmethoxy, phenoxy, propenyloxy or $C_{1-4}$alkyl substituted with up to 4 halo atoms; $R^1$ is hydrogen or $C_{1-4}$alkyl; and $R^6$ and $R^7$ are, each independently hydrogen, $C_{1-4}$alkyl, phenylmethyl or 2-propenyl or $R^6$ and $R^7$ combined with the nitrogen bearing said $R^6$ and $R^7$ may form a pyrrolidinyl piperidinyl or 4-morpholinyl radical.

**7.** A chemical compound according to claim 6 wherein Ar is a radical of formula (a- 2) wherein $R^{3'}$ is trifluoromethyl substituted at the meta position and $R^4$ and $R^5$ are each independently hydrogen, methyl, methoxy, halo, hydroxy, nitro, amino, trifluoromethyl, phenylmethoxy, phenoxy or propenyloxy.

**8.** A chemical compound according to claim 3 wherein the compound is trans-3-hydroxy-N,N,γ-trimethyl-α,α-diphenyl-4-[[3-(trifluoromethyl)benzoyl]amino]-1-piperidinebutanamide.

**9.** The use according to claim 1 of a compound as claimed in any of claims 3 to 8.

**10.** A pharmaceutical composition comprising a suitable pharmaceutical carrier and as active ingredient a therapeutically effective amount of a compound as claimed in any one of claims 3 to 8.

**11.** An anti-diarrheal composition comprising an inert carrier and as active ingredient an anti-diarrheal effective amount of at least one compound having the formula (I) as defined in claims 1 or 2 or of formula (I') as claimed in any of claims 3 to 8.

**12.** A method of preparing a pharmaceutical composition, characterized in that a therapeutically effective amount of a compound as defined in any of claims 3 to 8 is intimately mixed with suitable pharmaceutical carriers.

**13.** A compound as claimed in any one of claims 3 to 8 for use as a medicine.

**14.** A compound as claimed in any one of claims 3 to 8 for use as an anti-diarrheal medicine.

**15.** A process for preparing a chemical compound as claimed in any one of claims 3 to 8, characterized by

1) reacting a piperidine of formula

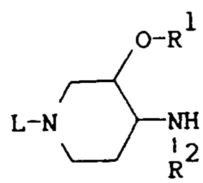

(II)

with a carboxylic acid of formula

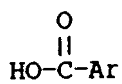

(III)

or a functional derivative thereof, in a reaction-inert solvent;

2) reacting a 7-oxa-3-azabicyclo[4.1.0]heptane of formula

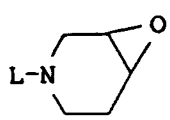

(IV)

with an wide of formula

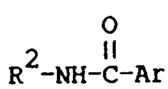

(V)

in a reaction-inert solvent; thus yielding a compound of formula

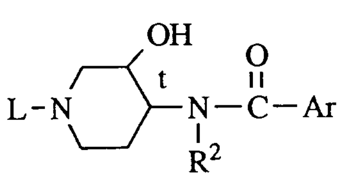

(I-a-1),

3) reacting a piperidone of formula

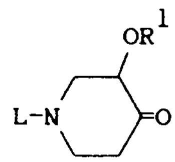

(VII)

with an amide of formula

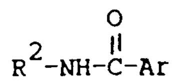

(V)

in a reductive medium; thus yielding a compound of formula

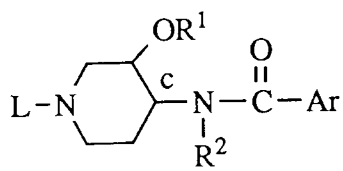

(I-b).

4) N-alkylating a piperidine of formula

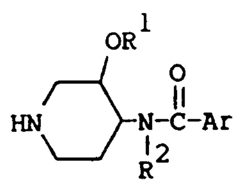

(VIII)

with a reagent of formula

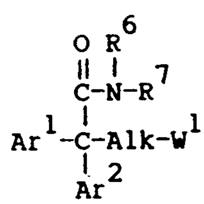

(IX)

wherein $W^1$ represents a reactive leaving group, or with a reagent of formula

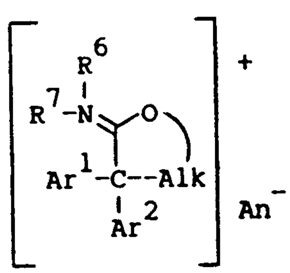

(X)

wherein $An^-$ represents an anion, in a reaction-inert solvent; wherein L represents a radical of formula

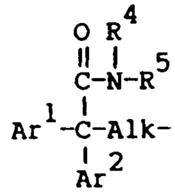

and, if desired, converting the compounds into each other following art-known functional group transformation procedures; and if further desired, converting the compounds of formula (I) into a therapeutically active non-toxic acid-addition salt form by treatment with an appropriate acid or, conversely, converting the acid-addition salt into the free base form with alkali, and/or preparing stereochemically isomeric forms thereof.

**Patentansprüche**

**1.** Verwendung einer Verbindung mit der Formel

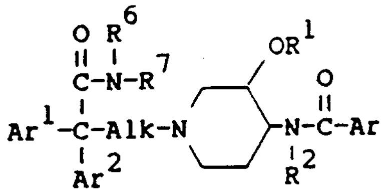

(I),

einer N-Oxidform, eines pharmazeutisch annehmbaren Säureadditionssalzes oder einer möglichen stereoisomeren Form hievon, worin

$R^1$ für Wasserstoff, $C_{1-6}$-Alkyl, Aryl-$C_{1-6}$-alkyl, $C_{1-6}$-Alkyl-carbonyl, Amino-$C_{1-6}$-alkyl oder Mono- oder Di($C_{1-6}$-alkyl)amino-$C_{1-6}$-alkyl steht;

$R^2$ Wasserstoff oder $C_{1-6}$-Alkyl bedeutet;

Ar Thienyl, Halogenthienyl, Furanyl, Halogenfuranyl, Pyridinyl, Aminopyridinyl, Thiazolyl, Imidazolyl oder einen Rest der Formel

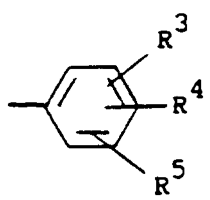

(a-1)

bedeutet, worin $R^3$, $R^4$ und $R^5$ jeweils unabhängig voneinander aus der aus Wasserstoff, $C_{1-6}$-Alkyl, $C_{1-6}$-Alkyloxy, Halogen, Hydroxy, Cyano, Nitro, Amino, Mono- und Di($C_{1-6}$-alkyl)amino, Aminocarbonyl, Arylcarbonylamino, $C_{1-6}$-alkylcarbonylamino, $C_{1-6}$-Alkylcarbonyl, $C_{1-6}$-Alkylcarbonyloxy, Aminosulfonyl, $C_{1-6}$-Alkylsulfinyl, $C_{1-6}$-Alkylsulfonyl, $C_{1-6}$-Alkylthio, Mercapto, $C_{3-6}$-Alkinyloxy, $C_{3-6}$-Alkenyloxy, Aryl-$C_{1-6}$-alkyloxy, Aryloxy und durch bis zu 4 Halogenatome substituiertem $C_{1-6}$-Alkyl bestehenden Gruppe ausgewählt sind;

Alk für $-CH_2-CH_2-$ oder $-CH_2-CH(CH_3)-$ steht;

$Ar^1$ und $Ar^2$ jeweils unabhängig voneinander Phenyl oder Halogenphenyl bedeuten;

$R^6$ und $R^7$ jeweils unabhängig voneinander Wasserstoff, $C_{1-6}$-Alkyl, Phenylmethyl oder 2-Propenyl darstellen, oder $R^6$ und $R^7$, zusammen mit dem diese Reste $R^6$ und $R^7$ tragenden Stickstoffatom, einen Pyrrolidinyl-, Piperidinyl-, $C_{1-6}$-Alkylpiperidinyl-, 4-Morpholinyl- oder 2,6-Di($C_{1-6}$-alkyl)-4-morpholinylrest ausbilden können;

worin Aryl für Phenyl steht, das gegebenenfalls durch bis zu 3 Substituenten substituiert ist, die jeweils unabhängig voneinander unter Halogen, Hydroxy, $C_{1-6}$-Alkyl, $C_{1-6}$-Alkyloxy, Aminosulfonyl, $C_{1-6}$-Alkylcarbonyl, Nitro, Trifluormethyl, Amino, Aminocarbonyl und Phenylcarbonyl, welcher Phenylcarbonylrest gegebenfalls durch bis zu 3 Halogen atome substituiert ist, ausgewählt sind; oder für Thienyl steht, das gegebenfalls durch Halogen oder $C_{1-16}$-Alkyl substituiert ist;

zur Herstellung eines Medikaments für die Behandlung von Diarrhoe.

2. Verwendung nach Anspruch 1, worin die Substituenten in 3- und 4-Stellung des Piperidinringes die trans-Konfiguration aufweisen.

3. Chemische Verbindung mit der Formel

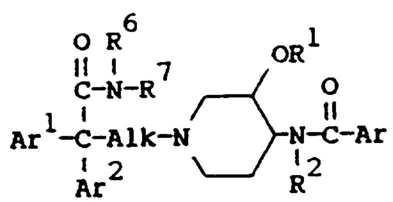

(I').

eine N-Oxidform, ein pharmazeutisch annehmbares Säureadditionssalz oder eine mögliche stereoisomere Form hievon, worin

$R^1$, $R^2$, $R^6$, $R^7$, Ar, $Ar^1$, $Ar^2$ und Alk wie in Anspruch 1 definiert sind, mit der Maßgabe, daß Ar eine andere Bedeutung als Phenyl oder 4-Amino-5-chlor-2-methoxyphenyl aufweist, wenn $R^6$ und $R^7$ beide Methyl bedeuten.

4. Chemische Verbindung nach Anspruch 3, worin Ar für Thienyl, Halogenthienyl, Furanyl, Halogenfuranyl, Pyridinyl, Aminopyridinyl, Thiazolyl, Imidazolyl oder einen Rest der Formel

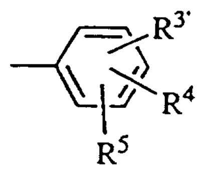

(a-2)

steht, worin $R^{3'}$ Aryl-$C_{1-6}$-alkyloxy, Aryloxy, $C_{3-6}$-Alkenyloxy, $C_{3-6}$-Alkinyloxy oder durch bis zu 4 Halogenatome substituiertes $C_{1-6}$-Alkyl darstellt.

5. Chemische Verbindung nach einem der Ansprüche 3 oder 4, worin die Substituenten in der 3- und 4-Stellung des Piperidinringes die trans-Konfiguration aufweisen.

6. Chemische Verbindung nach Anspruch 5, worin Ar für Thienyl, Halogenthienyl, Furanyl, Halogenfuranyl, Pyridinyl, Aminopyridinyl, Thiazolyl, Imidazolyl oder einen Rest der Formel (a-2) steht, worin $R^{3'}$ Phenylmethoxy, Phenoxy, Propenyloxy oder durch bis zu 4 Halogenatome substituiertes $C_{1-4}$-Alkyl bedeutet und $R^4$ und $R^5$ jeweils unabhängig voneinander Wasserstoff, $C_{1-4}$-Alkyl, $C_{1-4}$-Alkyloxy, Halogen, Hydroxy, Nitro, Amino, Phenylmethoxy, Phenoxy, Propenyloxy oder durch bis zu 4 Halogenatome substituiertes $C_{1-4}$-Alkyl bedeuten; $R^1$ Wasserstoff oder $C_{1-4}$-Alkyl darstellt; und $R^6$ und $R^7$ jeweils unabhängig voneinander Wasserstoff, $C_{1-4}$-Alkyl, Phenylmethyl oder 2-Propenyl bedeuten, oder $R^6$ und $R^7$, gemeinsam mit dem diese Reste $R^6$ und $R^7$ tragenden Stickstoff, einen Pyrrolidinyl-, Piperidinyl- oder 4-Morpholinylrest ausbilden können.

7. Chemische Verbindung nach Anspruch 6, worin Ar einen Rest der Formel (a-2) bedeutet, worin $R^{3'}$ in meta-Stellung substituiertes Trifluormethyl bedeutet, und $R^4$ und $R^5$ jeweils unabhängig voneinander Wasserstoff, Methyl, Methoxy, Halogen, Hydroxy, Nitro, Amino, Trifluormethyl, Phenylmethoxy, Phenoxy oder Propenyloxy bedeuten.

**8.** Chemische Verbindung nach Anspruch 3, worin die Verbindung trans-3-Hydroxy-N,N,γ-trimethyl-α,α-diphenyl-4-[[3-(trifluormethyl)benzoyl]amino]-1-piperidinbutanamid ist.

**9.** Verwendung einer Verbindung, wie in einem der Ansprüche 3 bis 8 beansprucht, gemäß Anspruch 1.

**10.** Pharmazeutische Zusammensetzung, umfassend einen geeigneten pharmazeutischen Träger und als wirksamen Bestandteil eine therapeutisch wirksame Menge einer Verbindung, wie in eine der Ansprüche 3 bis 8 beansprucht.

**11.** Anti-Diarrhoezusammensetzung, umfassend einen inerten Träger und als wirksamen Bestandteil eine anti-diarrhoeisch wirksame Menge wenigstens einer Verbindung mit der Formel (I), wie in den Ansprüchen 1 oder 2 definiert, oder der Formel (I'), wie in einem der Ansprüche 3 bis 8 definiert.

**12.** Verfahren zur Herstellung einer pharmazeutischen Zusammensetzung, dadurch gekennzeichnet, daß eine therapeutisch wirksame Menge einer Verbindung, wie in einem der Ansprüche 3 bis 8 definiert, innig mit geeigneten pharmazeutischen Trägen vermischt wird.

**13.** Verbindung nach einem der Ansprüche 3 bis 8 zur Verwendung als eine Medizin.

**14.** Verbindung nach einem der Ansprüche 3 bis 8 zur Verwendung als eine Anti-Diarrhoemedizin.

**15.** Verfahren zur Herstellung einer chemischen Verbindung nach einem der Ansprüche 3 bis 8, gekennzeichnet durch

1) Umsetzen eines Piperidins der Formel

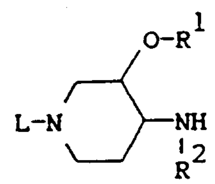

(II)

mit einer Carbonsäure der Formel

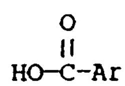

(III)

oder einem funktionellen Derivat hievon in einem reaktionsinerten Lösungsmittel;

2) Umsetzen eines 7-Oxa-3-azabicyclo[4.1.0]heptans der Formel

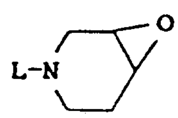

(IV)

mit einem Amid der Formel

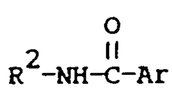

(V)

in einem reaktionsinerten Lösungsmittel, unter Ausbildung einer Verbindung der Formel

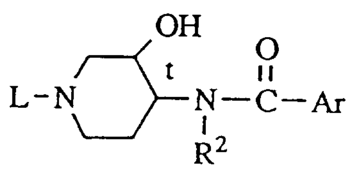

(I-a-1) ;

3) Umsetzen eines Piperidons der Formel

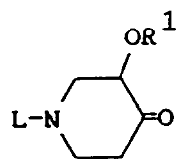

(VII)

mit einem Amid der Formel

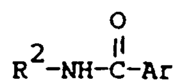

(V)

in einem reduzierenden Medium, unter Ausbildung einer Verbindung der Formel

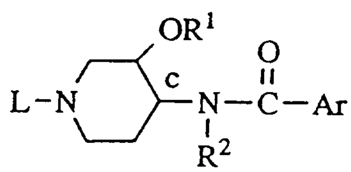

(I-b) ;

4) N-Alkylieren eines Piperidins der Formel

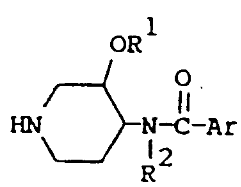

(VIII)

mit einem Reagens der Formel

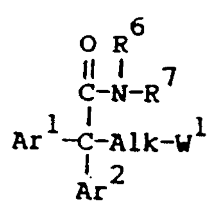

(IX) ,

worin $W^1$ eine reaktionsfähige Leaving-Gruppe bedeutet, oder mit einem Reagens der Formel

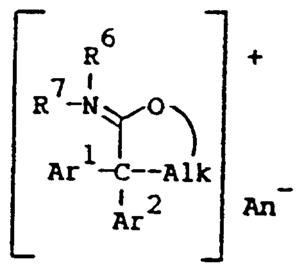

(X) ,

worin $An^-$ ein Anion darstellt, in einem reaktionsinerten Lösungsmittel; wobei L einen Rest der Formel

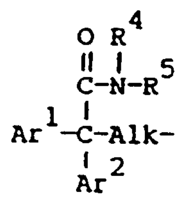

darstellt;

und gewünschtenfalls Überführen der Verbindungen ineinander nach bekannten funktionellen Gruppentransformationsmethoden; und, falls weiter gewünscht, Überführen der Verbindungen der Formel (I) in eine therapeutisch wirksame, nicht-toxische Säureadditionssalzform durch Behandlung mit einer geeigneten Säure, oder umgekehrt Umwandeln des Säureadditionssalzes mit Alkali in die freie Basenform, und/oder Bereiten stereochemisch isomerer Formen hievon.

## Revendications

**1.** Utilisation pour la préparation d'un médicament pour le traitement de la diarrhée, d'un composé répondant à la formule

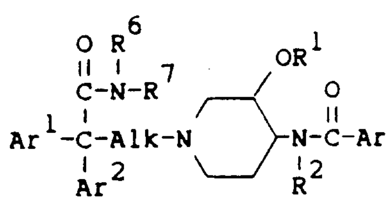

(I),

d'une de ses formes N-oxyde, d'un de ses sels d'addition d'acides pharmaceutiquement acceptables ou d'une de ses formes stéréoisomères possibles, dans laquelle

$R^1$ représente un atome d'hydrogène ou un groupe alkyle en $C_{1-6}$, aryl(alkyle en $C_{1-6}$), (alkyle en $C_{1-6}$)carbonyle, aminoalkyle en $C_{1-6}$ ou mon-ou di-(alkyle en $C_{1-6}$)aminoalkyle en $C_{1-6}$ ;

$R^2$ représente un atome d'hydrogène ou un groupe alkyle en $C_{1-6}$ ;

Ar représente un groupe thiényle, halogénothiényle, furanyle, halogénofuranyle, pyridinyle, aminopyridinyle, thiazolyle, imidazolyle ou un radical de formule :

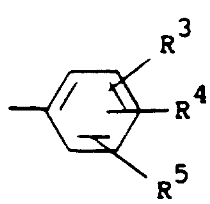

(a-1)

dans laquelle $R^3$, $R^4$ et $R^5$ sont chacun indépendamment choisis dans le groupe comprenant l'atome d'hydrogène et les groupes alkyle en $C_{1-6}$, alkyloxy en $C_{1-6}$, halogéno, hydroxyle, cyano, nitro, amino, mono- et di(alkyle en $C_{1-6}$)amino, aminocarbonyle, arylcarbonylamino, (alkyle en $C_{1-6}$)carbonylamino, (alkyle en $C_{1-6}$)carbonyle, (alkyle en $C_{1-6}$)carbonyloxy, aminosulfonyle, (alkyle en $C_{1-6}$)sulfinyle, (alkyle en $C_{1-6}$)sulfonyle, alkylthio en $C_{1-6}$, mercapto, alcynyloxy en $C_{3-6}$, alcényloxy en $C_{3-6}$, aryl-(alkyle en $C_{1-6}$)oxy, aryloxy et alkyle en $C_{1-6}$ substitué avec jusqu'à 4 atomes d'halogène ; Alk représente $-CH_2-CH_2-$ ou $-CH_2-CH(CH_3)-$ ;

$Ar^1$ et $Ar^2$ représentent, chacun de manière indépendante, un groupe phényle ou halogénophényle ;

$R^6$ et $R^7$ représentent, chacun de manière indépendante, un atome d'hydrogène, un groupe alkyle en $C_{1-6}$, phénylméthyle ou 2-propényle ou $R^6$ et $R^7$, combinés avec l'atome d'azote portant lesdits $R^6$ et $R^7$, peuvent former un radical pyrrolidinyle, pipéridinyle, (alkyle en $C_{1-6}$)pipéridinyle, 4-morpholinyle ou 2,6-di(alkyle en $C_{1-6}$)-4-morpholinyle ;

ledit groupe aryle étant un groupe phényle éventuellement substitué avec jusqu'à 3 trois substituants, chacun choisi de manière indépendante dans le groupe comprenant les atomes d'halogène et les groupes hydroxyle, alkyle en $C_{1-6}$, alkyloxy en $C_{1-6}$, aminosulfonyle, (alkyle en $C_{1-6}$)carbonyle, nitro, trifluorométhyle, amino, aminocarbonyle et phénylcarbonyle, ledit groupe phénylcarbonyle étant éventuellement substitué avec jusqu'à 3 atomes d'halogène ; ou un groupe thiényle éventuellement substitué par l' atome d'halogène ou le groupe alkyle en $C_{1-6}$.

2. Utilisation selon la revendication 1, dans laquelle les substituants en positions 3 et 4 du noyau pipéridine possèdent la configuration trans.

3. Composé chimique répondant à la formule

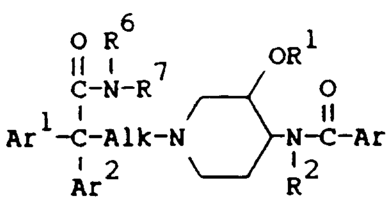

(I').

une de ses formes N-oxyde, un de ses sels d'addition d'acides pharmaceutiquement acceptables ou une de ses formes stéréoisomères possibles, dans laquelle $R^1$, $R^2$, $R^6$, $R^7$, Ar, $Ar^1$, $Ar^2$ et Alk sont tels que définis dans la revendication 1, à la condition que Ar soit différent d'un groupe phényle ou 4-amino-5-chloro-2-méthoxyphénylelorsque $R^6$ et $R^7$ sont tous les deux un groupe méthyle.

4. Composé chimique selon la revendication 3, dans lequel Ar représente un groupe thiényle, halogénothiényle, furanyle, halogénofuranyle, pyridinyle, aminopyridinyle, thiazolyle, imidazolyle ou un radical de formule

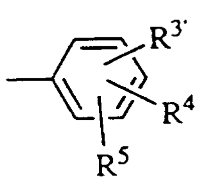

(a-2)

dans laquelle $R^{3'}$ représente un groupe aryl(alkyle en $C_{1-6}$)oxy, aryloxy, alcényloxy en $C_{3-6}$, alcynyloxy en $C_{3-6}$ ou alkyle en $C_{1-6}$ substitué avec jusqu'à 4 atomes d'halogène.

5. Composé chimique selon l'une quelconque des revendications 3 ou 4, dans lequel les substituants en positions 3 et 4 du noyau pipéridine ont la configuration trans.

6. Composé chimique selon la revendication 5, dans lequel Ar est un groupe thiényle, halogénothiényle, furanyle, halogénofuranyle, pyridinyle, aminopyridinyle, thiazolyle, imidazolyle ou un radical de formule

(a-2) dans laquelle $R^{3'}$ est un groupe phénylméthoxy, phénoxy, propényloxy ou alkyle en $C_{1-4}$ substitué par jusqu'à 4 atomes d'halogène et $R^4$ et $R^5$ représentent chacun, de manière indépendante, un atome d'hydrogène ou un groupe alkyle en $C_{1-4}$, alkyloxy en $C_{1-4}$, halogéno, hydroxyle, nitro, amino, phénylméthoxy, phénoxy, propényloxy ou alkyle en $C_{1-4}$ substitué par jusqu'à 4 atomes d'halogène ; $R^1$ est un atome d'hydrogène ou un groupe alkyle en $C_{1-4}$ ; et $R^6$ et $R^7$ représentent, chacun de manière indépendante, un atome d'hydrogène ou un groupe alkyle en $C_{1-4}$, phénylméthyle ou 2-propényle ou $R^6$ et $R^7$, combinés avec l'atome d'azote portant lesdits $R^6$ et $R^7$, peuvent former un radical pyrrolidinyle, pipéridinyle ou 4-morpholinyle.

**7.** Composé chimique selon la revendication 6, dans lequel Ar est un radical de formule (a-2) dans laquelle $R^{3'}$ est un groupe trifluorométhyle substitué en position meta et $R^4$ et $R^5$ sont chacun, de manière indépendante, un atome d'hydrogène ou un groupe méthyle, méthoxy, halogéno, hydroxyle, nitro, amino, trifluorométhyle, phénylméthoxy, phénoxy ou propényloxy.

**8.** Composé chimique selon la revendication 3, dans lequel le composé est le trans-3-hydroxy-N,N,γ-triméthyl-α,-α-diphényl-4-((3-(trifluorométhyl)benzoyl)amino)-1-pipéridinebutanamide.

**9.** Utilisation selon la revendication 1, d'un composé selon l'une quelconques des revendications 3 à 8.

**10.** Composition pharmaceutique comprenant un véhicule pharmaceutique convenable et à titre de principe actif, une quantité thérapeutiquement efficace d'un composé selon l'une quelconque des revendications 3 à 8.

**11.** Composition antidiarrhéïque comprenant un véhicule inerte, et à titre de principe actif, une quantité efficace du point de vue antidiarrhéïque d'au moins un composé répondant à la formule (I) tel que défini dans les revendications 1 ou 2 ou de formule (I') telle que définie dans l'une quelconque des revendications 3 à 8.

**12.** Procédé de préparation d'une composition pharmaceutique caractérisé en ce que l'on mélange intimement une quantité thérapeutiquement efficace d'un composé tel que défini dans l'une quelconque des revendications 3 à 8, avec des véhicules pharmaceutiques convenables.

**13.** Composé selon l'une quelconque des revendications 3 à 8 à utiliser comme médicament.

**14.** Composé selon l'une quelconque des revendications 3 à 8 à utiliser comme médicament antidiarrhéïque.

**15.** Procédé pour la préparation d'un composé chimique selon l'une quelconque des revendications 3 à 8, caractérisé par

1) la réaction d'une pipéridine de formule

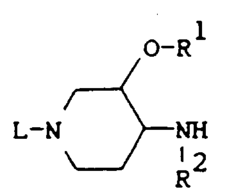

(II)

avec un acide carboxylique de formule

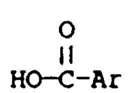

(III)

ou un de ses dérivés fonctionnels, dans un solvant inerte vis-à-vis de la réaction ;

2) la réaction d'un 7-oxa-3-azabicyclo(4.1.0)heptane de formule

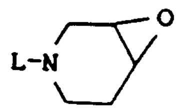

(IV)

avec un amide de formule

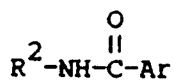

(V)

dans un solvant inerte vis-à-vis de la réaction ; ce qui donne ainsi un composé de formule

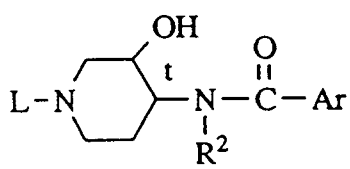

(I-a-1),

3) la réaction d'une pipéridone de formule

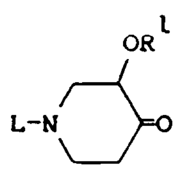

(VII)

avec un amide de formule

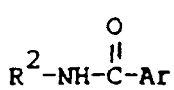

(V)

dans un milieu réductif ; ce qui donne ainsi un composé de formule

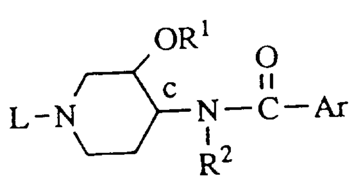

(I-b).

4) la N-alkylation d'une pipéridine de formule

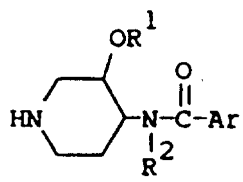

(VIII)

avec un réactif de formule

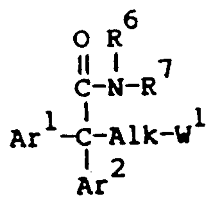

(IX)

dans laquelle $W^1$ représente un groupe partant réactif, ou avec un réactif de formule

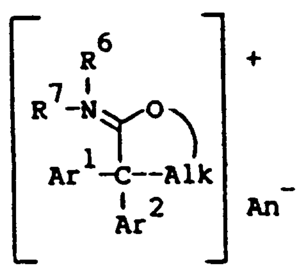

(X)

dans laquelle $An^-$ représente un anion, dans un solvant inerte vis-à-vis de la réaction ;
où L représente un radical de formule

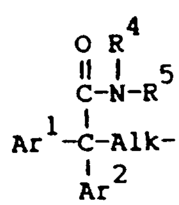

et si nécessaire, la conversion des composés les uns en les autres, en suivant les modes opératoires de transformation de groupes fonctionnels connus dans la technique ; et si on le souhaite encore, la conversion des composés de formule (I) en une forme de sel d'addition d'acide , non toxique, thérapeutiquement active, par traitement avec un acide approprié, ou inversement, la conversion du sel d'addition d'acide en la forme base libre avec un alcali, et/ou la préparation de leurs formes stéréochimiquement isomères.